(19) Europäisches Patentamt
European Patent Office
Office européen des brevets

(11)  **EP 2 651 467 B1**

(12)  **EUROPÄISCHE PATENTSCHRIFT**

(45) Veröffentlichungstag und Bekanntmachung des Hinweises auf die Patenterteilung:
**18.05.2016  Patentblatt 2016/20**

(21) Anmeldenummer: **11799369.1**

(22) Anmeldetag: **14.12.2011**

(51) Int Cl.:
***A61M 1/30*** *(2006.01)*

(86) Internationale Anmeldenummer:
**PCT/EP2011/006312**

(87) Internationale Veröffentlichungsnummer:
**WO 2012/079755 (21.06.2012 Gazette 2012/25)**

(54)  **EXTRAKORPORALE BLUTBEHANDLUNGSVORRICHTUNG**

EXTRACORPOREAL BLOOD TREATMENT APPLIANCE

SYSTÈME DE TRAITEMENT DE SANG EXTRACORPOREL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.12.2010  EP 10015621**

(43) Veröffentlichungstag der Anmeldung:
**23.10.2013  Patentblatt 2013/43**

(73) Patentinhaber: **B. Braun Avitum AG**
**34212 Melsungen (DE)**

(72) Erfinder:
• **MEIBAUM, Jörn**
  **34225 Baunatal (DE)**
• **BOCK, Gerhard**
  **36289 Friedewald (DE)**

(74) Vertreter: **Winter, Brandl, Fürniss, Hübner, Röss, Kaiser, Polte - Partnerschaft mbB Patent- und Rechtsanwaltskanzlei Bavariaring 10 80336 München (DE)**

(56) Entgegenhaltungen:
**DE-A1- 3 422 375      US-A- 4 596 550**
**US-A- 4 643 714      US-A- 5 318 511**

**Beschreibung**

Die Erfindung betrifft eine extrakorporale Blutbehandlungsvorrichtung gemäß dem Oberbegriff des Patentanspruchs 1.

Eine solche Vorrichtung ist beispielsweise aus der DE 34 22 375 A1 bekannt. Die dortige Vorrichtung weist einen Single-Lumen Katheter auf, durch welchen die extrakorporale Blutbehandlungsvorrichtung an den Blutkreislauf eines zu therapierenden Patienten anschließbar ist. Durch den einlumigen Anschluss des Patienten an die Vorrichtung ist es notwendig, dass darüber sowohl die Blutentnahme als auch das Reinfundieren des Blutes über diesen einlumigen Anschluss erfolgt.

Bei der DE 3422375 A1 sind mehrere Möglichkeiten der extrakorporalen Anordnung von Schläuchen, Drucksensoren, Klemmen und Pumpen zu sehen. Entscheidend für all diese Anordnungen ist, dass sie zum einen einen Arterienleitungsbehälter nach einer arteriellen Schlauchklemme und zum anderen einen Venenleitungsbehälter vor einer venösen Schlauchklemme aufweisen. Beide Behälter bilden einen Blutspeicher, so dass auch bei Verschluss einer der beiden Schlauchklemmen kontinuierlich Blut über den Dialysator gefördert werden kann. Die Steuerung zwischen arterieller Phase, in welcher dem Patienten das Blut entnommen und der Blutbehandlungsvorrichtung zugeführt wird, und venöser Phase, in welcher dem Patienten das entnommene Blut reinfundiert wird, geschieht in Abhängigkeit eines arteriellen und eines venösen Drucksensors, welche an dem Arterienleitungsbehälter bzw. Venenleitungsbehälter angebracht sind.

Dabei ist während der arteriellen Phase die arterielle Schlauchklemme geöffnet und die venöse geschlossen. Eine Blutpumpe fördert dabei das zu reinigende Blut durch das Schlauchsystem in den Dialysator und weiter in den Venenleitungsbehälter gegen die geschlossene venöse Schlauchklemme. Sobald in dem Venenleitungsbehälter der Druck eine vorgegebenen oberen Grenzwert erreicht hat, wird die arterielle Phase beendet und die venöse Phase begonnen, indem die venöse Schlauchklemme geöffnet und die arterielle geschlossen wird. Durch die Blutpumpe wird nun Blut aus dem Arterienleitungsbehälter in den Dialysator und weiter in den Venenleitungsbehälter gepumpt, während aus dem Venenleitungsbehälter gereinigtes Blut in den Patienten reinfundiert wird. Dies geschieht solange bis der sich in dem Arterienleitungsbehälter aufbauende Unterdruck einen unteren Grenzwert erreicht hat. Nun wird wiederum die arterielle Schlauchklemme geöffnet und die venöse geschlossen, so dass nunmehr die venöse Phase beendet ist und eine neue Phase, bestehend aus erneuter arterieller und erneuter venöser Phase, beginnt.

Eine weitere Vorrichtung wird in dem US-Patent US 4,643,714 beschrieben. Diese ist mit der zuvor in DE 3422375 A1 beschriebenen Vorrichtung in ihrem Aufbau prinzipiell identisch. So wird auch hier eine extrakorporale Anordnung von Schläuchen, Drucksensoren, Klemmen und einer Pumpe offenbart, bei der analog ein Arterienleitungsbehälter nach einer arteriellen Schlauchklemme und zum anderen ein Venenleitungsbehälter vor einer venösen Schlauchklemme vorliegt. Beide Behälter bilden auch hier einen Blutspeicher.
Die Steuerung zwischen arterieller Phase, in welcher dem Patienten das Blut entnommen und der Blutbehandlungsvorrichtung zugeführt wird, und venöser Phase, in welcher dem Patienten das entnommene Blut reinfundiert wird, geschieht hier in Abhängigkeit eines arteriellen Drucksensors am Arterienleitungsbehälter, um von der venösen Phase auf die arterielle Phase umzuschalten, und anders als in der DE 3422375 A1, in Abhängigkeit des gemessenen Hubvolumens, um von der arteriellen Phase auf die venöse Phase umzuschalten. Des Weiteren umfasst diese Vorrichtung einen Schutzmechanismus, bei welchem die Pumpe in der arteriellen Phase beim Erreichen eines bestimmten Überdruckes im Venenleitungsbehälter abgeschaltet wird, um eine Beschädigung am Dialysator zu vermeiden.

Das US-Patent US 5,318,511 beschreibt hingegen ein System einer extrakorporalen Anordnung von Schläuchen, Drucksensoren, Blutspeicherbehältern und Pumpen, bei dem die Steuerung zwischen arterieller Phase und venöser Phase über eine Druckmessung am Venenleitungsbehälter (Übergang von arterieller Phase zu venöser Phase) und eine fest definierte Pumpendrehzahl (Übergang von venöser zu arterieller Phase) geregelt ist. Jedoch ist das hierbei beschriebene Verfahren deutlich komplexer und es werden bis zu vier Pumpen benötigt, wobei zwei Pumpen jeweils für die arterielle bzw. venöse Phase zugeteilt werden. Eine Regelung oder Steuerung zur automatischen Flussregelung bei auftretenden Störungen ist in diesem US-Patent jedoch nicht offenbart.

Grundsätzlich arbeiten die in den Druckschriften DE 3422375 A1, US 4643714 und US 518511 beschriebenen Vorrichtungen bzw. die dort beschriebenen Verfahren zufriedenstellend, solange keine Störungen im extrakorporalen Kreislauf, insbesondere im Schlauchsystem, auftreten. Auf eine Störung im System und eine automatische Störungsbehebung unter der Fortführung einer kontinuierlichen Blutbehandlung wird in den zuvor beschriebenen Vorrichtungen nicht eingegangen und dies wird auch nicht weiter berücksichtigt. Lediglich offenbart US 4,643,714 ein automatisches Abstellen der Pumpe bei Totalausfall des Systems, um den Dialysator zu schützen.

Die im Stand der Technik beschriebenen Vorrichtungen sind jedoch nicht in der Lage, das Phasenvolumen über die Förderate der Pumpe automatisch bei Störungen anzupassen, um den Betrieb der Vorrichtung aufrecht zu erhalten. Eine solche Störung kann einerseits beispielweise ein Knick in der arteriellen bzw. venösen Schlauchleitung darstellen. Andererseits kann eine solche Störung auch in einem falschen Einstellen der Blutpumpengeschwindigkeit durch das Bedienpersonal seine Ursache haben, wobei unter einer solchen Störung auch der Start der Therapie angesehen werden soll. Solche Störungen können im Extremfall dazu führen, dass kein effektiver Blutfluss - aus dem Patientenkörper heraus und wieder hinein stattfindet bzw. mehr stattfindet. Im Folgenden sei eine. solche Störung beispielhaft kurz anhand eines Knickes in der venösen Schlauchleitung beschrieben:

Durch einen Knick in der venösen Schlauchleitung während der venösen Phase erhöht sich dort der Flusswiderstand, wodurch der Abfluss des gereinigten Blutes in den Patienten abnimmt und sich der Venenleitungsbehälter entsprechend langsamer entleert. Dies hat zur Folge, dass sich das arterielle Phasenvolumen über mehrere Phasen verkleinert, weil bei gleichem Blutfluss über den Dialysator bis zum Erreichen des unteren Druckgrenzwertes in dem Arterienleitungsbehälter sich der Venenleitungsbehälter nicht entsprechend entleert hat. Demzufolge wird der obere Druckgrenzwert in dem Venenleitungsbehälter während der folgenden Phasen immer früher erreicht. Das Gesamtsystem beginnt demzufolge zwischen arterieller und venöser Phase zu oszillieren, ohne dass ein effektiver Blutfluss durch die extrakorporale Blutreinigungsmaschine stattfindet. Daher erfolgt in solchen Situationen, insbesondere auch unter Berücksichtigung des weiter unten beschriebenen Totvolumens, kein Reinfundieren bereits gereinigten Blutes in den Patienten. Somit findet in diesen Situationen mit der in DE 3422375 A beschriebenen extrakorporalen Blutreinigungsmaschine effektiv keine Therapie mehr statt. Dies kann nur unterbunden werden, wenn das Bedienpersonal regulierend eingreift.

Die Folgen eines Knicks in der venösen Schlauchleitung sei auch im Folgenden an der Vorrichtung aus US 4643714 geschildert. Durch die Behinderung des Blutflusses in der venösen Schlauchleitung während der venösen Phase kann der Venenleitungsbehälter nur unzureichend entleert werden und mit laufender Pumpe und stetiger Blutzuführung aus dem Dialysator, steigt der Druck im Venenleitungsbehälter rasch an. Dieser wird jedoch für einen Wechsel zur arteriellen Phase nicht berücksichtigt und der Wechsel erfolgt erst ab einem gewissen Unterdruck im Arterienleitungsbehälter. Die Venenleitung wird daraufhin geschlossen und die Arterienleitung geöffnet. Da das angestaute Flüssigkeitsvolumen im Venenleitungsbehälter während der venösen Phase nicht abfließen konnte, wird der gesetzte Grenzwert für den Druck des Venenleitungsbehälters mit der laufenden Pumpe umgehend erreicht. Mit Erreichen des Grenzwertes wird die Pumpe abgeschaltet und der Arterienleitungsbehälter füllt sich ohne eine Pumpleistung aufgrund des dort vorliegenden Unterdrucks. Ist eine definierte Menge an Blut in den Arterienleitungsbehälter nachgeflossen und entspricht diese Menge einem gesetzten Grenzwert für das Hubvolumen, so wird die Pumpe automatisch aktiviert und es findet unter Schließung der Arterienleitung und Öffnen der Venenleitung ein Wechsel in die venöse Phase statt. Es hat sich somit ein kritischer Zustand eingestellt, in welchem der Venenleitungsbehälter als auch der Arterienleitungsbehälter vollständig gefüllt sind, und zudem eine Behinderung der Venenleitung vorliegt. Eine Regelung der Pumpleistung in der venösen Phase existiert nicht und mit laufender Pumpe und wachsendem Druck im Venenleitungsbehälter eskaliert das System schließlich, da ein erneuter Wechsel in die arterielle Phase erst mit einem gewissen Unterdruck im Arterienleitungsbehälter verbunden ist. Dies kann nur unterbunden werden, wenn das Bedienpersonal regulierend eingreift.

Auch die in US 4596550 beschriebene Vorrichtung könnte einer Störung, z.B. einem Knick in der Venenleitung, nicht automatisch entgegenwirken. Durch die Behinderung des Blutflusses in der venösen Schlauchleitung würde es umgehend zu einer Eskalation des Systems kommen, da zwischen dem Venenleitungsbehälter und dem Patientenzugang eine zusätzliche Pumpe geschaltet ist, welche für den Pumpvorgang des dialysierten Blutes vom Venenleitungsbehälter zum Patienten verantwortlich ist. Da es nach dieser Pumpe keine Sensorik für den Flusswiderstand gibt, kann die Pumpleistung auch nicht den gegebenen Verhältnissen angepasst werden. Eine Eskalation des Systems kann auch hier nur unterbunden werden, wenn das Bedienpersonal regulierend eingreift.

Bei den letzten beiden Beispielen ist somit hervorzuheben, dass es den Vorrichtungen an einem Regelsystem mangelt, dass besonders während einer venösen Phase einer Störung entgegenwirkt.

Es ist daher Aufgabe der Erfindung, eine Vorrichtung nach dem Oberbegriff des Patentanspruchs 1 zur Verfügung zu stellen, mit welcher trotz auftretender Störungen im Blutflusssystem die Therapie fortgesetzt werden kann und auch bei Behebung oder Wegfall der Störung die Vorrichtung automatisch wieder zu einer effektiveren Leistung übergehen kann.

Beschreibung

Gelöst wird diese Aufgabe durch eine Vorrichtung mit den Merkmalen des Patentanspruchs 1. Vorteilhafte Ausgestaltungen der Erfindung finden sich in den Unteransprüchen, den Beispielen und den Figuren.

Im Gegensatz zu dem zuvor zitierten Stand der Technik zeichnet sich die vorliegende Erfindung durch ein automatisiertes Regelsystem aus, in welchem, abhängig vom momentan geförderten Blutvolumen einer Phase (arteriell oder venös) und dessen Abweichung von einem definierten Sollwert ($V_{soll}$), eine Störung selbständig erkannt und durch kontinuierliche Anpassung der Pumpenleistung (Hoch- als auch Runter-Regelung), der Betrieb der Vorrichtung zur extrakorporalen Blutbehandlung aufrecht erhalten werden kann, indem der Sollwert des Phasenvolumens ($V_{soll}$) dem Istwert des Phasenvolumens ($V_{ist}$) durch Anpassung der Förderrate $Q_{BP}$ der mindestens einen Pumpe (40) mittels einer Steuereinrichtung (50) angeglichen wird.

Wie hierin verwendet, haben folgende Begriffe die folgenden Bedeutungen:

| | |
|---|---|
| $t_{Phase}$: | Ist die Dauer einer Phase vom öffnen der Arterienleitungs-Schließeinrichtung 22 bis zum nächsten Öffnen der Arterienleitungs-Schließeinrichtung 22. |
| $t_{Art\_Phase}$: | Ist die Dauer der arteriellen Phase vom Öffnen der Arterienleitungs-Schließeinrichtung 22 bis zum Schließen der Arterienleitungs-Schließeinrichtung 22. |
| $t_{Ven\_Phase}$: | Ist die Dauer der venösen Phase vom Öffnen der Venenleitungs-Schließeinrichtung 32 bis zum Schließen Venenleitungs-Schließeinrichtung 32. |
| $V_{Phase}$: | Phasenvolumen: Ist das während der Dauer einer Phase durch die Pumpe geförderte Volumen. |
| $V_{ist}$: | Istwert des Phasenvolumens: Der Istwert des Phasenvolumens ist das während einer Phase tatsächlich geförderte Phasenvolumen, welches bei einem einwandfreien Dialyseverlauf dem Sollwert des Phasenvolumens entspricht und bei auftretenden Störungen vom Sollwert des Phasenvolumens abweicht. |
| $V_{soll}$: | Sollwert des Phasenvolumens: Der Sollwert des Phasenvolumens wird für die erste Phase bei Beginn der Dialyse vorgegeben, z.B. durch das Bedienpersonal oder automatisch festgelegt aufgrund von Referenzwerten früherer Dialysesitzungen und kann durchaus auch variieren. Für jede weitere Phase entspricht der Sollwert des Phasenvolumens dem Istwert des Phasenvolumens der vorhergehenden Phase. |
| $\Delta V$: | Differenz zwischen dem Istwert des Phasenvolumens und dem Sollwert des Phasenvolumens. Ist $\Delta V > 0$ so wurde in der aktuellen Phase ein größeres Phasenvolumen gefördert als in der vorherigen Phase. Ist $\Delta V < 0$ so wurde in der aktuellen Phase ein kleineres Phasenvolumen gefördert als in der vorherigen Phase. |
| $Q_{BP}$: | Ist die Förderrate der Pumpe (40) angegeben in mL/s. |

Die vorliegende Erfindung betrifft somit eine extrakorporale Blutbehandlungsvorrichtung mit

- einer Steuereinrichtung (50) zur Steuerung des Öffnens und Schließens einer Arterienleitung (2) bei gleichzeitigem oder im wesentlichen gleichzeitigem Schließen und Öffnen einer Venenleitung (3) in einer Single-Lumen-Anordnung (10), mit welcher unbehandeltes Blut durch die Single-Lumen-Anordnung (10) und die Arterienleitung (2) abgezogen, durch eine Blutbehandlungseinrichtung (5) geleitet und behandeltes Blut durch die Venenleitung (3) und die Single-Lumen-Anordnung (10) zurückgeführt wird,
- einem Arterienleitungsbehälter (20), der in der Arterienleitung (2) vor der Blutbehandlungseinrichtung (5) angeordnet ist,
- einem Venenleitungsbehälter (30), der in der Venenleitung (3) hinter der Blutbehandlungseinrichtung (5) angeordnet ist,
- einer Sensoreinrichtung (31) zum Erfassen einer über einem Grenzwert liegenden Menge behandelten Bluts in dem Venenleitungsbehälter (30),
- einer Sensoreinrichtung (21) zum Erfassen einer unter einem Grenzwert liegenden Menge unbehandelten Bluts in dem Arterienleitungsbehälter (20),
- einer Arterienleitungs-Schließeinrichtung (22) zum Verschließen der Arterienleitung (2) bei gleichzeitigem oder im wesentlichen gleichzeitigem Öffnen der Venenleitung (3) ansprechend auf ein Signal A, das von der Sensoreinrichtung (31) an die Arterienleitungs-Schließeinrichtung (22) übermittelt wird,
- einer Venenleitungs-Schließeinrichtung (32) zum Verschließen der Venenleitung (3) bei gleichzeitigem oder im wesentlichen gleichzeitigem Öffnen der Arterienleitung (2) ansprechend auf ein Signal B, das von der Sensoreinrichtung (21) an eine Venenleitungs-Schließeinrichtung (32) übermittelt wird und
- wenigstens einer Pumpe (40) zur Förderung eines Phasenvolumens $V_{Phase}$ mit einer Förderrate $Q_{BP}$,

dadurch gekennzeichnet, dass
die Steuereinrichtung (50) dann, wenn das tatsächlich geförderte Phasenvolumen $V_{ist}$ von dem Phasenvolumens $V_{soll}$ abweicht, die Förderrate $Q_{BP}$ der wenigstens einen Pumpe (40) so regelt, dass diese Abweichung $\Delta V$ gegen Null geht.

Dass der Arterienleitungsbehälter (20) in der Arterienleitung (2) vor der Blutbehandlungseinrichtung (5) angeordnet ist, bedeutet, dass sich der Arterienleitungsbehälter (20) in Blutflussrichtung vor der Blutbehandlungseinrichtung (5) befindet und somit zwischen Arterienleitungs-Schließeinrichtung (22) und Blutbehandlungseinrichtung (5) angeordnet ist.

In sämtlichen Ausführungen der hierin offenbarten extrakorporalen Blutbehandlungsvorrichtung kann das Merkmal

>> - einem Arterienleitungsbehälter (20), der in der Arterienleitung (2) vor der Blutbehandlungseinrichtung (5) angeordnet ist, <<

wie folgt ersetzt werden durch:

>> - einem Arterienleitungsbehälter (20), der in der Arterienleitung (2) in Blutflussrichtung vor der Blutbehandlungseinrichtung (5) angeordnet ist, << oder durch

>> - einem Arterienleitungsbehälter (20), der in der Arterienleitung (2) zwischen Arterienleitungs-Schließeinrichtung (22) und Blutbehandlungseinrichtung (5) angeordnet ist, <<

Vorzugsweise ist der Arterienleitungsbehälter (20) zwischen Arterienleitungs-Schließeinrichtung (22) und Pumpe (40) angeordnet.

Dass der Venenleitungsbehälter (30) in der Venenleitung (3) hinter der Blutbehandlungseinrichtung (5) angeordnet ist, bedeutet, dass sich der Venenleitungsbehälter (30) in Blutflussrichtung hinter der Blutbehandlungseinrichtung (5) befindet und somit zwischen Venenleitungs-Schließeinrichtung (32) und Blutbehandlungseinrichtung (5) angeordnet ist.

In sämtlichen Ausführungen der hierin offenbarten extrakorporalen Blutbehandlungsvorrichtung kann das Merkmal

>> - einem Venenleitungsbehälter (30), der in der Venenleitung (3) hinter der Blutbehandlungseinrichtung (5) angeordnet ist, <<

wie folgt ersetzt werden durch:

>> - einem Venenleitungsbehälter (30), der in der Venenleitung (3) in Blutflussrichtung hinter der Blutbehandlungseinrichtung (5) angeordnet ist, << oder durch
>> - einem Venenleitungsbehälter (30), der in der Venenleitung (3) zwischen Venenleitungs-Schließeinrichtung (32) und Blutbehandlungseinrichtung (5) angeordnet ist, <<

Der Begriff "gleichzeitig" oder "im wesentlichen gleichzeitig" bedeutet, dass das Schließen der Arterienleitungs-Schließeinrichtung (22) zeitgleich mit dem Öffnen der Venenleitungs-Schließeinrichtung (32) erfolgt und entsprechend das Schließen der Venenleitungs-Schließeinrichtung (32) zeitgleich mit dem Öffnen der Arterienleitungs-Schließeinrichtung (22) stattfindet. Somit wird ein kontinuierlicher Blutfluß aufrecht erhalten. Vorzugsweise bezeichnet der Begriff "zeitgleich", dass beide Vorgänge, d.h. Schließen von 32 und Öffnen von 22 als auch Schließen von 22 und Öffnen von 32 innerhalb einer Sekunde ablaufen.

Das Merkmal

- einer Sensoreinrichtung (31) zum Erfassen einer über einem Grenzwert liegenden Menge behandelten Bluts in dem Venenleitungsbehälter (30),

bedeutet, dass eine Obergrenze für enthaltenes behandeltes Blut oder ein Füllstand oder ein mit dem Füllstand korrelierender Maximaldruck detektiert werden sollen, wodurch angegeben wird, dass der Venenleitungsbehälter (30) eine vorgegebene maximale Flüssigkeitsmenge enthält und damit voll ist und nun die Entleerung eingeleitet werden soll.

Somit läßt sich das vorgenannte Merkmal bei allen hierin offenbarten Ausführungsformen ersetzen durch:

>> - einer Sensoreinrichtung (31) zum Erfassen des Füllstandes in dem Venenleitungsbehälter (30), << oder
>> - einer Sensoreinrichtung (31) zum Erfassen eines maximalen Füllstandes in dem Venenleitungsbehälter (30), << oder
>> - einer Sensoreinrichtung (31) zum Erfassen, ob ein vorgegebener Füllstand in dem Venenleitungsbehälter (30) erreicht worden ist, << oder
>> - einer Sensoreinrichtung (31) zum Erfassen eines maximalen Drucks in dem Venenleitungsbehälter (30), << oder
>> - einer Sensoreinrichtung (31) zum Erfassen, ob ein vorgegebener Druck in dem Venenleitungsbehälter (30) erreicht worden ist, <<.

Das Merkmal

- einer Sensoreinrichtung (21) zum Erfassen einer unter einem Grenzwert liegenden Menge unbehandelten Bluts in dem Arterienleitungsbehälter (20),

bedeutet, dass eine Untergrenze für enthaltenes unbehandeltes Blut oder ein Füllstand oder ein mit dem Füllstand korrelierender Minimaldruck oder Unterdruck detektiert werden sollen, wodurch angegeben wird, dass der Arterienleitungsbehälter (20) eine vorgegebene minimale Flüssigkeitsmenge enthält und damit leer ist und nun die Befüllung eingeleitet werden soll.

Somit läßt sich das vorgenannte Merkmal bei allen hierin offenbarten Ausführungsformen ersetzen durch:

>> - einer Sensoreinrichtung (21) zum Erfassen des Füllstandes in dem Arterienleitungsbehälter (20), << oder

>> - einer Sensoreinrichtung (21) zum Erfassen eines minimalen Füllstandes in dem Arterienleitungsbehälter (20), << oder

>> - einer Sensoreinrichtung (21) zum Erfassen, ob ein vorgegebener Füllstand in dem Arterienleitungsbehälter (20) erreicht worden ist, << oder

>> - einer Sensoreinrichtung (21) zum Erfassen eines Minimaldrucks oder Unterdrucks in dem Arterienleitungsbehälter (20), << oder

>> - einer Sensoreinrichtung (21) zum Erfassen, ob ein vorgegebener Druck oder Unterdruck in dem Arterienleitungsbehälter (20) erreicht worden ist, <<.

Das Merkmal

- einer Arterienleitungs-Schließeinrichtung (22) zum Verschließen der Arterienleitung (2) bei gleichzeitigem oder im wesentlichen gleichzeitigem Öffnen der Venenleitung (3) ansprechend auf ein Signal A, das von der Sensoreinrichtung (31) an die Arterienleitungs-Schließeinrichtung (22) übermittelt wird,

bedeutet, dass die Sensoreinrichtung (31) wie oben beschrieben einen Füllstand oder maximalen Füllstand oder einen vorgegebenen Druck bzw. einen vorgegebenen Überdruck misst oder das Erreichen eines solchen vorgegebenen Grenzwertes überprüft und bei Erreichung des vorgegebenen Füllstandes oder Druckes ein Signal an die Steuereinrichtung (50) sendet, welche dann das Schließen der Arterienleitungs-Schließeinrichtung (22) und zeitgleiches Öffnen der Venenleitungs-Schließeinrichtung (32) regelt.

Obiges Merkmal kann daher durch eine der folgenden Formulierungen ersetzt werden:

- einer Arterienleitungs-Schließeinrichtung (22) zum Verschließen der Arterienleitung (2) bei zeitgleichem Öffnen der Venenleitung (3) ansprechend auf ein Signal A, das von der Sensoreinrichtung (31) über die Steuereinrichtung (50) an die Arterienleitungs-Schließeinrichtung (22) übermittelt wird,
oder
- einer Arterienleitungs-Schließeinrichtung (22) zum Verschließen der Arterienleitung (2) bei zeitgleichem Öffnen der Venenleitung (3) ansprechend auf ein Signal A, das von der Steuereinrichtung (50) an die Arterienleitungs-Schließeinrichtung (22) übermittelt wird.

Das Merkmal

- einer Venenleitungs-Schließeinrichtung (32) zum Verschließen der Venenleitung (3) bei gleichzeitigem oder im wesentlichen gleichzeitigem Öffnen der Arterienleitung (2) ansprechend auf ein Signal B, das von der Sensoreinrichtung (21) an eine Venenleitungs-Schließeinrichtung (32) übermittelt wird

bedeutet, dass die Sensoreinrichtung (21) wie oben beschrieben einen Füllstand oder minimalen Füllstand oder einen vorgegebenen Druck bzw. einen vorgegebenen Unterdruck misst oder das Erreichen eines solchen vorgegebenen Grenzwertes überprüft und bei Erreichung des vorgegebenen Füllstandes oder Druckes ein Signal an die Steuereinrichtung (50) sendet, welche dann das Schließen der Venenleitungs-Schließeinrichtung (32) und zeitgleiches Öffnen der Arterienleitungs-Schließeinrichtung (22) regelt.

Obiges Merkmal kann daher durch eine der folgenden Formulierungen ersetzt werden:

- einer Venenleitungs-Schließeinrichtung (32) zum Verschließen der Venenleitung (3) bei zeitgleichem Öffnen der Arterienleitung (2) ansprechend auf ein Signal B, das von der Sensoreinrichtung (21) über die Steuereinrichtung (50) an eine Venenleitungs-Schließeinrichtung (32) übermittelt wird,
oder
- einer Venenleitungs-Schließeinrichtung (32) zum Verschließen der Venenleitung (3) bei zeitgleichem Öffnen der Arterienleitung (2) ansprechend auf ein Signal B, das von der Steuereinrichtung (50) an eine Venenleitungs-Schließeinrichtung (32) übermittelt wird.

Die vorliegende Erfindung betrifft somit eine extrakorporale Blutbehandlungsvorrichtung mit

- einer Steuereinrichtung (50) zur Steuerung des Öffnens und Schließens einer Arterienleitung (2) bei zeitgleichem Schließen und Öffnen einer Venenleitung (3), mit welcher unbehandeltes Blut durch die Arterienleitung (2) abziehbar ist, durch eine Blutbehandlungseinrichtung (5) leitbar und als behandeltes Blut durch die Venenleitung (3) zurückführbar ist,
- einem Arterienleitungsbehälter (20), der in der Arterienleitung (2) vor der Blutbehandlungseinrichtung (5) angeordnet ist,
- einem Venenleitungsbehälter (30), der in der Venenleitung (3) hinter der Blutbehandlungseinrichtung (5) angeordnet ist,
- einer Sensoreinrichtung (31) zum Erfassen des Füllstandes an behandeltem Blut in dem Venenleitungsbehälter (30),
- einer Sensoreinrichtung (21) zum Erfassen des Füllstandes an unbehandelten Bluts in dem Arterienleitungsbehälter (20),
- einer Arterienleitungs-Schließeinrichtung (22) zum Verschließen der Arterienleitung (2) bei zeitgleichem Öffnen der Venenleitung (3) ansprechend auf ein Signal A, das von der Sensoreinrichtung (31) über die Steuereinrichtung (50) an die Arterienleitungs-Schließeinrichtung (22) übermittelt wird,
- einer Venenleitungs-Schließeinrichtung (32) zum Verschließen der Venenleitung (3) bei zeitgleichem Öffnen der Arterienleitung (2) ansprechend auf ein Signal B, das von der Sensoreinrichtung (21) über die Steuereinrichtung (50) an eine Venenleitungs-Schließeinrichtung (32) übermittelt wird und
- wenigstens einer Pumpe (40) zur Förderung eines Phasenvolumens $V_{Phase}$ mit einer Förderrate $Q_{BP}$,

dadurch gekennzeichnet, dass
die Steuereinrichtung (50) dann, wenn das tatsächlich geförderte Phasenvolumen $V_{ist}$ von dem Phasenvolumens $V_{soll}$ abweicht, die Förderrate $Q_{BP}$ der wenigstens einen Pumpe (40) so regelt, dass diese Abweichung $\Delta V$ gegen Null geht.

Anders formuliert betrifft die vorliegende Erfindung eine extrakorporale Blutbehandlungsvorrichtung mit

- einer Steuereinrichtung (50) zur Steuerung des Öffnens und Schließens einer Arterienleitung (2) bei zeitgleichem Schließen und Öffnen einer Venenleitung (3), mit welcher unbehandeltes Blut durch die Arterienleitung (2) abziehbar ist, durch eine Blutbehandlungseinrichtung (5) leitbar und als behandeltes Blut durch die Venenleitung (3) zurückführbar ist,
- einem Arterienleitungsbehälter (20), der in der Arterienleitung (2) vor der Blutbehandlungseinrichtung (5) angeordnet ist,
- einem Venenleitungsbehälter (30), der in der Venenleitung (3) hinter der Blutbehandlungseinrichtung (5) angeordnet ist,
- einer Sensoreinrichtung (31) zum Erfassen des Füllstandes an behandeltem Blut in dem Venenleitungsbehälter (30),
- einer Sensoreinrichtung (21) zum Erfassen des Füllstandes an unbehandelten Bluts in dem Arterienleitungsbehälter (20),
- einer Arterienteitungs-Schließeinrichtung (22) zum Verschließen der Arterienleitung (2) bei zeitgleichem Öffnen der Venenleitung (3) ansprechend auf ein Signal A, das von der Sensoreinrichtung (31) über die Steuereinrichtung (50) an die Arterienleitungs-Schließeinrichtung (22) übermittelt wird,
- einer Venenleitungs-Schließeinrichtung (32) zum Verschließen der Venenleitung (3) bei zeitgleichem Öffnen der Arterienleitung (2) ansprechend auf ein Signal B, das von der Sensoreinrichtung (21) über die Steuereinrichtung (50) an eine Venenleitungs-Schließeinrichtung (32) übermittelt wird und
- wenigstens einer Pumpe (40) zur Förderung eines vordefinierten Phasenvolumens $V_{soll}$ mit einer Förderrate $Q_{BP}$ während einer Phase mit einer Phasendauer $t_{Phase}$

dadurch gekennzeichnet, dass
die Steuereinrichtung (50) dann, wenn das tatsächlich geförderte Phasenvolumen $V_{ist}$ von dem Phasenvolumens $V_{soll}$ innerhalb einer Phase abweicht, die Förderrate $Q_{BP}$ der wenigstens einen Pumpe (40) während der nachfolgenden Phase so regelt, dass diese Abweichung $\Delta V$ gegen Null geht.

Der vorgenannte kennzeichnende Teil kann auch alternativ wie folgt formuliert werden:

dadurch gekennzeichnet, dass
die Steuereinrichtung (50) dann, wenn das tatsächlich geförderte Phasenvolumen $V_{ist}$ von dem Phasenvolumens $V_{soll}$ innerhalb einer Phase abweicht, die Förderrate $Q_{BP}$ der wenigstens einen Pumpe (40) während der nachfolgenden Phase so regelt, dass $V_{ist}$ an $V_{soll}$ angeglichen wird.

oder

dadurch gekennzeichnet, dass

die Steuereinrichtung (50) dann, wenn das tatsächlich geförderte Phasenvolumen $V_{ist}$ von dem Phasenvolumens $V_{soll}$ innerhalb einer Phase abweicht, die Förderrate $Q_{BP}$ der wenigstens einen Pumpe (40) so regelt, dass $V_{ist}$ an $V_{soll}$ angeglichen wird,

oder

dadurch gekennzeichnet, dass

die Steuereinrichtung (50) ausgebildet ist, um ein Phasenvolumen in Abhängigkeit der aktuellen Förderrate $Q_{BP}$ der wenigstens einen Pumpe (40) während einer Phase selbsttätig auf einen vordefinierten Wert $V_{soll}$ einzustellen, wobei

- selbige Steuereinrichtung (50) als Regelkreis ausgebildet ist, bei dem das Phasenvolumen auf einen definierbaren Sollwert $V_{soll}$ regelbar ist, wobei die Abweichung $\Delta V$ eines Istwertes $V_{ist}$ des Phasenvolumens von dessen Sollwert $V_{soll}$ zur Regelung verwendet wird, und wobei
- die Regelung mit einer Änderung der Leistung der Pumpe (40) eine selbsttätige Angleichung des Istwertes $V_{ist}$ des Phasenvolumens an dessen Sollwert $V_{soll}$ und der einhergehenden Änderung der Zeitspanne einer Phase $t_{Phase}$ beinhaltet, wobei besagte Regelung in der venösen Phase als auch in der arteriellen Phase erfolgen kann.

Der Sollwert des Phasenvolumens $V_{soll}$ der aktuellen Phase ist der Istwert des Phasenvolumens $V_{ist}$ der vorhergehenden Phase. Fließt das Blut ungehindert und ist keine Verengung in der Arterienleitung (2) und/oder der Venenleitung (3) so entspricht der Sollwert des Phasenvolumens $V_{soll}$ dem Istwert des Phasenvolumens $V_{ist}$ und $\Delta V$ ist Null oder bei Berücksichtigung eines gewissen Toleranzbereichs annähernd Null.

Tritt hingegen eine Verengung im Bereich der Arterienleitung (2) in dem Abschnitt zwischen Patient und dem Arterienleitungsbehälter (20) und/oder im Bereich der Venenleitung (3) in dem Abschnitt zwischen Venenleitungsbehälter (30) und Patient während einer Phase auf, so ist das tatsächlich geförderte Phasenvolumen $V_{ist}$ in der Phase, wo die Verengung aufgetreten ist, geringer als in der vorhergehenden Phase, d.h. $V_{soll}$ ist größer als $V_{ist}$ und damit $\Delta V = V_{ist} - V_{soll} < 0$. Somit wurde während der vorherigen Phase ein größeres Phasenvolumen gefördert als in der aktuellen Phase und das während der aktuellen Phase tatsächlich geförderte Phasenvolumen $V_{ist}$ wird für die nachfolgende Phase zum Sollwert des Phasenvolumens $V_{soll}$. Damit in der nachfolgenden Phase der Istwert des Phasenvolumens $V_{ist}$ den Sollwert des Phasenvolumens $V_{soll}$ erreicht, wird die Förderrate der Pumpe (40) so reduziert, dass in der nachfolgenden Phase das Volumen $\Delta V$ weniger gefördert wird. Erreicht in der nachfolgenden Phase der Istwert des Phasenvolumens $V_{ist}$ den Sollwert des Phasenvolumens $V_{soll}$ so bedeutet dies, dass keine weitere Verengung aufgetreten ist, die bestehende Verengung aber auch nicht aufgeweitet wurde oder sich aufgeweitet hat. Schreitet hingegen die Verengung weiter fort oder kommt eine weitere Verengung hinzu, so ist auch in der nachfolgenden Phase der Istwert des Phasenvolumens $V_{ist}$ kleiner als der Sollwert des Phasenvolumens $V_{soll}$ und $\Delta V$ ist wiederum kleiner Null.

Die erfindungsgemäße Vorrichtung in den hierin beschriebenen Ausführungsformen kann somit bei Verengungen im Bereich der Leitungen zwischen Venenleitungsbehälter (30), Patient und Arterienleitungsbehälter (20) den Blutfluß selbständig und automatisch regeln, um den Fortgang der Dialyse aufrecht zu erhalten und ohne die Dialysesitzung unterbrechen zu müssen oder durch einen Warnhinweis den Eingriff des Pflegepersonals erforderlich zu machen. Voraussetzung für diese automatische Regelung der Förderrate der Pumpe (40) ist, dass die Verengung in der Arterienleitung (2) zwischen Patient und Arterienleitungsbehälter (20) nicht derart stark ist, dass das Blut langsamer in den Arterienleitungsbehälter (20) fließt, als die Pumpe (40) es dem Arterienleitungsbehälter (20) wieder entnimmt. Dies wäre eine derart starke Störung, dass ein Warnhinweis ausgegeben würde und die Dialyse unterbrochen werden müsste. Vorzugsweise wird die Pumpe (40) dann automatisch ausgeschaltet. Gleiches gilt, wenn die Verengung in der Venenleitung (3) zwischen Venenleitungsbehälter (30) und Patient derart stark wäre, dass das Blut langsamer aus dem Venenleitungsbehälter (30) abfließen würde als die Pumpe (40) Blut in den Venenleitungsbehälter (30) nachliefert. Dies wäre ebenfalls ein Dauerstörzustand, der zur Ausgabe eines Warnhinweises und zur Abschaltung der Pumpe (40) führen würde.

Umfasst die erfindungsgemäße Vorrichtung mehr als eine Pumpe (40) so befindet sich die weitere Pumpe (40) oder die weiteren Pumpen (40) zwischen Dialysator (5) und Venenleitungsbehälter (30) oder zwischen Arterienleitungsbehälter (20) und Dialysator (5). Alle Pumpen (40) laufen dann vorzugsweise mit derselben Förderrate $Q_{BP}$ und werden simultan durch die Steuereinrichtung (50) geregelt.

Wird nun eine bestehende Verengung wieder erweitert, z.B. durch das Pflegepersonal oder auch von alleine durch Bewegung des Patienten, so führt dies dazu, dass während der aktuellen Phase ein größeres Phasenvolumen transportiert wird als bei der vorhergehenden Phase. Somit ist am Ende der aktuellen Phase der Sollwert des Phasenvolumens $V_{soll}$ geringer als der Istwert des Phasenvolumens $V_{ist}$ und damit ist $\Delta V > 0$. Dies führt nun dazu, dass automatisch die Förderrate $Q_{BP}$ während der nachfolgenden Phase um das Volumen $\Delta V$ gesteigert wird, damit der

Sollwert des Phasenvolumens $V_{soll}$ in der nachfolgenden Phase wieder dem Istwert des Phasenvolumens $V_{ist}$ in der nachfolgenden Phase entspricht.

Erfindungsgemäß ist somit, dass bei verlangsamten Abfluß des Blutes aus dem Venenleitungsbehälter (30) die Förderrate der Pumpe (40) automatisch reduziert werden kann und bei einem wieder verbesserten Abluß des Blutes aus dem Venenleitungsbehälter (30) die Förderrate der Pumpe (40) automatisch auch wieder gesteigert werden kann, ohne dass Bedienpersonal in die Steuerung eingreifen oder diese Nachregeln muss. Gleiches gilt natürlich auch für einen gestörten Zufluß des Blutes in den Arterienleitungsbehälter (20).

Eine weitere alternative Formulierung der Erfindung lautet wie folgt, wobei die Erfindung eine extrakorporale Blutbehandlungsvorrichtung betrifft mit

- einer Steuereinrichtung (50) zur Steuerung des Öffnens und Schließens einer Arterienleitung (2) bei gleichzeitigem Schließen und Öffnen einer Venenleitung (3) in einer Single-Lumen-Anordnung (10), mit welcher unbehandeltes Blut durch die Arterienleitung (2) abziehbar ist, durch eine Blutbehandlungseinrichtung (5) leitbar und behandeltes Blut durch die Venenleitung (3) zurückführbar ist,
  einem Arterienleitungsbehälter (20), der in der Arterienleitung (2) zwischen der Arterienleitungs-Schließeinrichtung (22) und der Blutbehandlungseinrichtung (5) angeordnet ist,
- einem Venenleitungsbehälter (30), der in der Venenleitung (3) zwischen der Blutbehandlungseinrichtung (5) und der Venenleitungs-Schließeinrichtung (32) angeordnet ist,
- einer Sensoreinrichtung (31) zum Erfassen des Füllstandes im Venenleitungsbehälter (30) und einer Signalweiterleitung an die Steuereinrichtung 50,
- einer Sensoreinrichtung (21) zum Erfassen des Füllstandes im Arterienleitungsbehälter (20) und einer Signalweiterleitung an die Steuereinrichtung 50,
- einer Arterienleitungs-Schließeinrichtung (22) zum Verschließen der Arterienleitung (2) bei gleichzeitigem Öffnen der Venenleitung (3) ansprechend auf ein Signal A, das von der Steuereinrichtung (50) an die Arterienleitungs-Schließeinrichtung (22) übermittelt wird, wenn der Füllstand des Venenleitungsbehälters (30) einen definierten Grenzwert erreicht hat,
- einer Venenleitungs-Schließeinrichtung (32) zum Verschließen der Venenleitung (3) bei gleichzeitigem Öffnen der Arterienleitung (2) ansprechend auf ein Signal B, das von der Steuereinrichtung (50) an eine Venenleitungs-Schließeinrichtung (32) übermittelt wird, wenn der Füllstand des Arterienleitungsbehälters (20) einen definierten Grenzwert erreicht hat, und
- wenigstens einer Pumpe (40) zur Förderung eines vordefinierten Phasenvolumens $V_{Phase}$ mit einer Förderrate $Q_{BP}$,

dadurch gekennzeichnet, dass
die Steuereinrichtung (50) dann, wenn das tatsächlich geförderte Phasenvolumen $V_{ist}$ von dem Phasenvolumen $V_{soll}$ mit einer Abweichung $\Delta V$ abweicht, die Förderrate $Q_{BP}$ der wenigstens einen Pumpe (40) so regelt, dass diese Abweichung $\Delta V$ gegen Null geht.

Erfindungsgemäß wird die Förderrate $Q_{BP}$ der Pumpe (40) oder der Pumpen (40) während einer laufenden Phase nicht verändert, sondern jeweils am Anfang einer nachfolgenden Phase, sofern sich bei der vorhergehenden Phase ein $\Delta V$ ungleich Null oder größer als ein vordefiniertes Toleranzintervall von z.B. 1 mL ergeben hat. Somit wird bei einem $\Delta V < 0$ die Förderrate der Pumpe (40) oder der Pumpen (40) am Anfang der nachfolgenden Phase um das Volumen $\Delta V$ reduziert, d.h. wenn davon ausgegangen wird, dass die Phasendauer der nachfolgende Phase der Phasendauer der aktuellen Phase entspricht, wird die Förderrate der Pumpe (40) oder der Pumpen (40) am Anfang der nachfolgenden Phase so reduziert, dass während der Phasendauer der nachfolgenden Phase insgesamt das Volumen $\Delta V$ weniger gefördert wird.

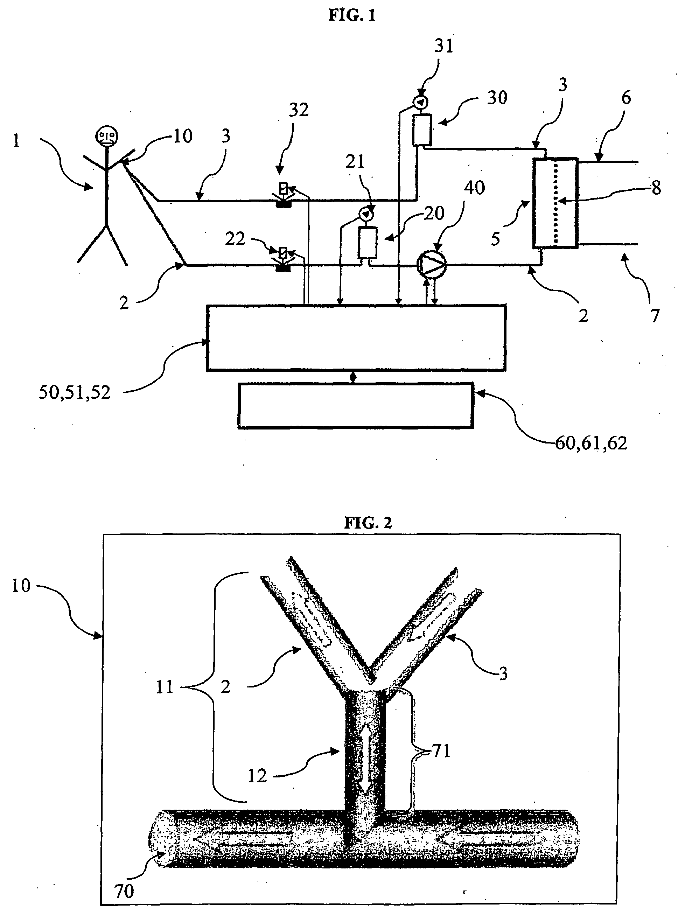

Erfindungsgemäß ist die Single-Lumen-Anordnung (10) in diesem Dokument definiert, wie insbesondere aus Figur 1 ersichtlich, als eine Anordnung von einem Patientenzugang (12) am Blutgefäßsystem (70) des Patienten (s. Fig. 2), mit einem Y-Stück (11) (s. Fig. 2), sowie der daran angeschlossenen Arterienleitung (2) und der Venenleitung (3), wobei die Arterienleitung (2) durch den Arterienleitungsbehälter (20) und die Pumpe (40) bis zur Blutbehandlungseinrichtung (5) führt, und die Venenleitung (3) von der Blutbehandlungseinrichtung (5) durch den Venenleitungsbehälter (30) über das Y-Stück (11) bis zum Patienten führt. Die Single-Lumen-Anordnung (10) kann dabei den Zugang zum Patienten beispielsweise mittels einer Nadel oder einer flexiblen Verweilkanüle realisieren.

Eine Phase umfasst dabei die Zeitspanne, welche zwischen dem Öffnen der Arterienleitungs-Schließeinrichtung (22) bis zum nächsten Öffnen der Arterienleitungs-Schließeinrichtung (22) liegt, und als Summe der arteriellen Phase und der venösen Phase definiert ist. Dabei beschreibt die arterielle Phase die Zeitspanne, welche zwischen dem Öffnen der Arterienleitungs-Schließeinrichtung (22) bis zum Schließen der Arterienleitungs-Schließeinrichtung (22) liegt, und die venöse Phase beschreibt die Zeitspanne, welche zwischen dem Öffnen der Venenleitungs-Schließeinrichtung (32) bis zum Schließen der Venenleitungs-Schließeinrichtung (32) liegt.

Die Vorrichtung ist dadurch gekennzeichnet, dass die Steuereinrichtung (50) als Regelkreis ausgebildet ist, bei dem die selbsttätige Angleichung des Istwertes $V_{ist}$ des Phasenvolumens an dessen Sollwert $V_{soll}$ und der einhergehenden Änderung der Zeitspanne einer Phase $t_{Phase}$ in der venösen Phase als auch in der arteriellen Phase erfolgen kann.

Sie ist darüber hinaus dadurch gekennzeichnet, dass der Regler des Regelkreises als P-Regler, I-Regler, D-Regler, PI-Regler, PD-Regler, PID-Regler, Zweipunktregler, Dreipunktregler, Mehrpunktregler bzw. Fuzzy-Regler ausgebildet ist.

Der Sollwert $V_{soll}$ des Phasenvolumens ist dabei mindestens fünfmal, vorzugsweise mindestens zehnmal so groß ist wie ein Totvolumen $V_{Tot}$ (71) der Single-Lumen-Anordnung (10).

Die Steuereinrichtung (50) ist derart ausgebildet, dass sie die Regelung auf das Phasenvolumen unmittelbar nach Detektion einer Abweichung $\Delta V \neq 0$ des Istwertes $V_{ist}$ des Phasenvolumens von dessen Sollwert $V_{soll}$ um eine vorgegebenen Totzeit $T_{Tot}$ verzögert, wobei die vorgegebenen Totzeit $T_{Tot}$ dabei wenigstens zwei Phasen, bevorzugt wenigstens vier Phasen und maximal zehn Phasen beträgt.

Die wenigstens eine Pumpe (40) ist zwischen dem Arterienleitungsbehälter (20) und dem Venenleitungsbehälter (30) angeordnet. Die Sensoreinrichtungen (21, 31) sind vorzugsweise als Drucksensoren ausgebildet und der durch die Sensoreinrichtung (31) gemessene Überdruck im Venenleitungsbehälter (30) löst ein Verschließen der Arterienleitungs-Schließeinrichtung (22) und ein Öffnen der Venenleitungs-Schließeinrichtung (32) aus und der durch die Sensoreinrichtung (21) gemessene Unterdruck löst im Arterienleitungsbehälter (20) ein Verschließen der Venenleitungs-Schließeinrichtung (32) und ein Öffnen der Arterienleitungs-Schließeinrichtung (22) aus.

Zur Bestimmung des Istwertes $V_{ist}$ des Phasenvolumens wird dabei die Förderrate $Q_{BP}$ der Pumpe (40) sowie die Zeitspanne einer Phase $t_{Phase}$ verwendet, wobei die Zeitspanne einer Phase $t_{phase}$ definiert ist als Summe der Zeitspanne $t_{Ven\_Phase}$, die zwischen dem Signal A und dem Signal B liegt, die von der Steuereinrichtung (50) an die Arterienleitungs-Schließeinrichtung (22) und die Venenleitungs-Schließeinrichtung (32) übermittelt werden, und der Zeitspanne $t_{Art\_Phase}$, die zwischen dem Signal B und dem Signal A liegt, die von der Steuereinrichtung (50) an die Arterienleitungs-Schließeinrichtung (22) und die Venenleitungs-Schließeinrichtung (32) übermittelt werden.

Bei den verwendeten Begriffen der arteriellen und venösen Phase wird die gleiche Definition zugrundegelegt, wie sie eingangs für die DE 3422375 A1 beschrieben sind, wobei hier anstatt arterieller und venöser Schlauchklemmen allgemeiner von Arterienleitungs- bzw. Venenleitungs-Schließeinrichtungen ausgegangen wird.

Für den effektiven Betrieb von Blutbehandlungsvorrichtungen ist von Bedeutung, dass das behandelte akkumulierte Blutvolumen so groß wie möglich ist. Die Besonderheit beim Einlumen- oder auch Single-Lumen-Betrieb reduziert ein Volumen, welches durch den einlumigen Anschluss der Blutbehandlungsvorrichtungen an das Gefäßsystem des Patienten definiert ist und als Totvolumen bezeichnet wird, das behandelte Blutvolumen. Da das bereits gereinigte Blut in dem einlumigen Zugang zum Patienten, also dem Totvolumen, in der arteriellen Phase wieder extrakorporal dem Dialysator zugeführt wird, sinkt die Effektivität der Behandlung. Das in diesem Totvolumen $V_{Tot}$ enthaltene Blut wird Rezirkulationsvolumen genannt, weil während jeder arteriellen Phase eine gewisse Blutmenge aufgrund des bestehenden Totraums im extrakorporalen Kreislauf nicht in den Patientenkörper reinfundiert wird. Dieses Totvolumen ist durch den einlumigen Patientenzugang an den sich ein Y-Stück zum Zusammenführen des arteriellen und venösen Schlauchsystems anschließt, bedingt. Grundsätzlich gilt, je hoher die Rezirkulation, desto geringer ist die Clearance-Leistung. Als Normbereich gilt ein Rezirkulationsanteil kleiner 10% des Phasenvolumens. Eine weitere Größe beim Einnadel-/Einlumen-Betrieb ist das Phasenvolumen $V_{Phase}$ Das Phasenvolumen $V_{Phase}$ ergibt sich aus dem Blutvolumen in der arteriellen Phase $V_{Art\_Phase}$, das dem Patienten entnommen wird, oder dem Blutvolumen in der venösen Phase $V_{Ven\_Phase}$, das dem Patienten wieder zurückgegeben wird. Das Blutvolumen der arteriellen und venösen Phase unterscheidet sich durch das Flüssigkeitsvolumen, das im Dialysator durch die Ultrafiltration dem Blut entzogen wird.

Das effektive dem Dialysator neu aus dem Patientenkörper während einer Phase zugeführte Blutvolumen $V_{eff}$ berechnet sich daher zu:

$$V_{eff} = V_{Art\_Phase} - V_{Tot}.$$

Die Ermittlung des Phasenvolumens $V_{phase}$ erfolgt durch Multiplikation der Förderrate $Q_{BP}$ der Blutpumpe und der Zeitspanne $t_{Phase}$ einer Phase zu:

$$V_{Phase} = Q_{BP} * t_{Phase}$$

wobei sich die Zeitspanne $t_{Phase}$ einer Phase aus der Addition der Zeitspanne der arteriellen Phase $t_{Art\_Phase}$ und der Zeitspanne der venösen Phase $t_{Ven-Phase}$ ergibt zu:

$$t_{Phase} = t_{Art\_Phase} + t_{Ven\_Phase}$$

Zu unterscheiden von der Förderrate $Q_{BP}$ der Blutpumpe sind der arterielle Fluss $Q_{Art\text{-}Blut}$, sowie der venöse Fluss $Q_{Ven\text{-}Blut}$, die ebenfalls entsprechend nachfolgender Gleichungen mit dem Phasenvolumen $Vp_{hase}$ zusammenwirken. Es gilt:

$$V_{Phase} = V_{Art\_Phase} + V_{Ven\_Phase},$$

wobei mit

$$V_{Art\_Phase} = Q_{Art\_Blut} * t_{Art\_Phase}$$

und

$$V_{Ven\_Phase} = Q_{Ven\_Blut} * t_{Ven\_Phase}$$

für $V_{Phase}$ folgender Zusammenhang gilt:

$$V_{Phase} = Q_{BP} * ( t_{Art\_Phase} + t_{Ven\_Phase} )$$

Das akkumulierte Blutvolumen das dem Patienten während der Therapie entnommen und wieder reinfundiert wird ergibt sich zu:

$$V_{Therapie} = Q_{Art\_Blut} * t_{Art\_Phase} * t_{Therapie} / ( t_{Art\_Phase} + t_{Ven\_Phase} )$$

während das gesamte Lotvolumen $V_{Tot\text{-}Gesamt}$ sich über die gesamte Therapie zu

$$V_{Tot\text{-}Gesamt} = V_{Tot} * t_{Therapie} / ( t_{Art\_Phase} + t_{Ven\_Phase} )$$

aufsummiert.

Für die effektive behandelte Blutmenge beim Einnadel-/Einlumen-Betieb hat es sich daher erfindungsgemäß als vorteilhaft erwiesen, das Phasenvolumen auf Grundlage von dem verwendeten Gefäßzugang vorzugeben und den Blutfluss kontinuierlich auf einen vordefinierten bzw. vorgegebenen Wert einzustellen. Damit ist sichergestellt, dass bei vorgegebenem Gefäßzugang der optimale Blutfluss erreicht wird, bei dem der Rezirkulationsanteil beispielsweise kleiner 10% ist. Damit verbessert diese Erfindung die Qualität der Therapie und trägt zur höchst möglichen behandelten Blutmenge bei. Realisiert wird dies dadurch, dass in der erfindungsgemäßen Vorrichtung eine Steuereinrichtung vorgesehen ist, mit welcher ein Phasenvolumen in Abhängigkeit der aktuellen Förderrate $Q_{BP}$ der wenigstens einen Blutpumpe während einer Phase selbsttätig auf einen vordefinierten bzw. vorgegebenen Wert $V_{soll}$ einstellbar ist.

Dazu wird das während einer Phase geförderte Blutvolumen $V_{ist}$ bestimmt und mit dem vordefinierten bzw. vorgegebenen Wert $V_{soll}$ verglichen. Sofern keine Differenz zwischen $V_{ist}$ und $V_{soll}$ festgestellt werden kann, läuft die Therapie wie in der Phase, für die zuletzt $V_{ist}$ bestimmt wurde, weiter. Wird allerdings eine Differenz $\Delta V = V_{soll} - V_{ist} \neq 0$ festgestellt, dann wird durch die Steuereinrichtung der erfindungsgemäßen Vorrichtung veranlasst, dass die Förderrate der Blutpumpe entsprechend geändert wird, so dass in der nachfolgenden Phase das Phasenvolumen in Richtung des vordefinierten bzw. vorgegebenen Wertes $V_{soll}$ beeinflusst wird.

Dabei ist zu berücksichtigen, dass sowohl die Arterienleitungs-Schließeinrichtung als auch die Venenleitungs-Schließeinrichtung immer noch bei Erreichen der durch die den Arterien- bzw. Venenleitungsbehälter zugeordneten Sensoreinrichtungen erfassten Grenzwerte geöffnet bzw. geschlossen werden. Liegt allerdings das während einer Phase geförderte Blutvolumen $V_{ist}$ unter dem Sollwert $V_{soll}$, wird entgegen der zu erwartenden Steigerung eine Senkung der Förderrate der Pumpe durchgeführt. Dies hat seine Ursache darin, dass das Nichterreichen des Sollwertes $V_{soll}$ nicht

in einer zu geringen Förderrate der Pumpe liegt, sondern daran, dass der vorgegebene Grenzwert im Arterien- bzw. Venenleitungsbehälter zu früh erreicht wurde und deshalb die arterielle bzw. venöse Phase beendet, obwohl das zu fördernde Blutvolumen $V_{soll}$ in dieser Phase noch nicht erreicht wurde. Deshalb wird erfindungsgemäß durch die Steuereinrichtung das Phasenvolumen in Abhängigkeit der aktuellen Förderrate $Q_{BP}$ der wenigstens einen Pumpe dahingehend auf einen vordefinierten Wert $V_{soll}$ eingestellt, dass durch eine Erniedrigung der Förderrate $Q_{BP}$ die Zeitspanne vergrößert wird, in der im Arterien- bzw. Venenleitungsbehälter der vorgegebene Grenzwert erreicht wird. In dieser nun vergrößerten Zeitspanne der arteriellen bzw. venösen Phase hat das System nun mehr Zeit den entsprechenden vordefinierten Wert $V_{soll}$ für das Phasenvolumen zu erreichen. Liegt in den darauffolgenden Phasen das Phasenvolumen immer noch unterhalb dieses vordefinierten Wertes $V_{soll}$ wird die Förderrate der Pumpe nochmals entsprechend gesenkt. Um eine entsprechend gleichmäßige Therapie zu erzielen, ist es natürlich auch möglich, die Förderrate $Q_{BP}$ der wenigstens einen Pumpe entsprechend zu erhöhen, wenn das während einer Phase geförderte Blutvolumen $V_{ist}$ über dem Sollwert $V_{soll}$ liegt.

Neben den bereits eingangs genannten Einflussgrößen wie Störungen im Schlauchsystem und in der Förderrate der Pumpe hat auch eine Viskositätsänderung des Blutes Auswirkungen auf das Phasenvolumen. Auch solche Störungen sind mit der erfindungsgemäßen Vorrichtung in ebenso einfacher Weise zu handhaben wie die Einstellung des Phasenvolumens mit Hilfe der erfindungsgemäßen Vorrichtung beim Starten einer Blutbehandlung bei einem Patienten.

In der Praxis hat es sich bewährt, die Steuereinrichtung als Regelkreis auszubilden, bei dem das Phasenvolumen selbsttätig auf einen Sollwert regelbar ist, wobei die Abweichung $\Delta V$ eines Istwertes $V_{ist}$ des Phasenvolumens von dessen Sollwert $V_{soll}$ zur Regelung verwendet wird. Vorteilhafterweise ist der Regler des Regelkreises als P-Regler, I-Regler, D-Regler, PI-Regler, PD-Regler, PID-Regler, Zweipunktregler, Dreipunktregler, Mehrpunktregler bzw. Fuzzy-Regler ausgebildet. Durch die große Auswahl dieser verschiedenen Reglertypen ist es in einfacher Weise möglich, die Regelung auch sehr fein abzustimmen, so dass bereits kleinste $\Delta V$-Werte zu einem Eingreifen der Steuerung führen und somit das Phasenvolumen über die gesamte Therapie annähernd konstant gehalten werden kann, wodurch eine optimale Therapie gewährleistet wird. Auf die einzelnen mathematischen Formulierungen der verschiedenen Regler wird an dieser Stelle verzichtet, da die Ausgestaltung eines entsprechenden Reglers und der dort zu verwendeten konstanten Faktoren je nach verwendeter erfindungsgemäßer Vorrichtung variieren kann und deren Bestimmung im Ermessen des Fachmanns liegt.

Weiterhin vorteilhaft ist es, den Sollwert $V_{soll}$ des Phasenvolumens mindestens fünfmal, vorzugsweise mindestens zehnmal so groß zu halten wie das Totvolumen der Single-Lumen-Anordnung 10. Dadurch ist sichergestellt, dass ein möglichst großes effektives Blutvolumen über die gesamte Therapie gereinigt wird.

In einer weiteren Ausführungsform der Erfindung hat es sich als sinnvoll erwiesen die Regelung des Phasenvolumens zeitverzögert durchzuführen. Dazu ist die Steuereinrichtung derart ausgebildet, dass sie die Regelung auf das Phasenvolumen unmittelbar nach Detektion einer Abweichung $\Delta V \neq 0$ des Istwertes $V_{ist}$ des Phasenvolumens von dessen Sollwert $V_{soll}$ um eine vorgegebene Totzeit $T_{Tot}$ verzögert. Durch diese Maßnahme ist vermieden, dass die Regelung unnötigerweise anspricht, wenn die Abweichung $\Delta V \neq 0$ innerhalb eines Toleranzbereichs liegt. Dies kann beispielsweise durch systembedingte Einflüsse, wie beispielsweise bei Verwendung einer konstruktionsbedingt nicht gleichmäßig fördernden Rollenpumpe, oder aber durch nur kurzzeitige Störungen, wie beispielsweise eines nur kurzzeitigen Knickes in der Arterien- und/oder Venenleitung, bedingt sein. Die Totzeit $T_{Tot}$ sollte deshalb nicht absolut festgelegt sein, sondern sich an einer bestimmten Anzahl vorgegebener Phasen, bestehend wie bereits zuvor beschrieben jeweils aus einer arteriellen und einer venösen Phase definieren.

In der Praxis hat es sich deshalb als vorteilhaft erwiesen, diese Totzeit $T_{Tot}$ wenigstens als zwei solcher Phasen, bevorzugt als vier solcher Phasen und maximal als zehn solcher Phasen zu definieren.

Um den apparativen Aufwand zu minimieren ist es vorteilhaft, nur eine Pumpe zu verwenden, die zwischen dem Arterienleitungsbehälter und dem Venenleitungsbehälter anzuordnen ist. Dadurch ist eine definierte Blutförderung sowohl in der arteriellen als auch in der venösen Phase mit nur einer Pumpe im System gewährleistet, was nicht nur im Hinblick auf Herstellungs- und Anschaffungskosten, sondern auch im Hinblick auf Wartungsarbeiten sehr ökonomisch ist. In einer besonderen Ausgestaltung der Erfindung hat es sich als vorteilhaft erwiesen, dass die den Arterienleitungs- und Venenleitungsbehälter zugeordneten Sensoreinrichtungen als Drucksensoren ausgebildet sind. Damit kann in einfacher Weise über auftretende Unter- bzw. Überdrücke im Arterienleitungs- bzw. Venenleitungsbehälter deren Füllstand und mit Hilfe der Steuereinrichtung bzw. der Förderrate der Pumpe und der zwischen Öffnen und Schließen vergangen Zeit bzw. des in dieser Zeit geförderten Blutes der Istwert $V_{ist}$ für das Phasenvolumen bestimmt werden.

Weitere Ziele, Vorteile, Merkmale und Anwendungsmöglichkeiten der vorliegenden Erfindung ergeben sich aus der nachfolgenden Beschreibung der Ausführungsbeispiele anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen und deren Rückbeziehung.

**Figurenbeschreibung**

Figur 1: eine schematische Darstellung eines Ausführungsbeispiels einer erfindungsgemäßen Vorrichtung mit bereits angeschlossenem Patienten,

Figur 2: eine schematische Darstellung eines Ausführungsbeispiels einer an ein Gefäßsystem eines Patienten angeschlossenen Single-Lumen-Anordnung 10,

Figur 3: Darstellung der Abhängigkeit des Phasenvolumens vom Fördervolumen der Blutpumpe für verschiedene Blutviskositäten,

Figur 4: Beispiel einer Signalschwankung des Phasenvolumens in Abhängigkeit von der Zeit bei Verwendung einer Rollenpumpe.

In Figur 1 ist ein Ausführungsbeispiel einer erfindungsgemäßen extrakorporalen Blutbehandlungsvorrichtung schematisch dargestellt. In dieser Darstellung ist ein zu therapierender Patient 1 durch eine Single-Lumen-Anordnung 10 an die Vorrichtung angeschlossen. Die Single-Lumen Anordnung 10, wie insbesondere aus Figur 1 und 2 ersichtlich, beinhaltet die Anordnung von einem Patientenzugang 12 am Blutgefäßsystem 70 des Patienten, mit einem Y-Stück 11, sowie der daran angeschlossenen Arterienleitung 2 und der Venenleitung 3, wobei die Arterienleitung 2 durch den Arterienleitungsbehälter 20 und die Pumpe 40 bis zur Blutbehandlungseinrichtung 5 führt, und die Venenleitung 3 durch den Venenleitungsbehälter 30 bis zur Blutbehandlungseinrichtung 5 führt.. Die Single-Lumen-Anordnung 10 kann dabei den Zugang zum Patienten beispielsweise mittels einer Nadel oder einer flexiblen Verweilkanüle realisieren.

Während der Therapie wird dem Gefäßsystem 70 des Patienten 1 mittels des Patientenzugangs 12 der Single-Lumen-Anordnung 10 Blut entnommen. Aus dem Patientenzugang 12 gelangt das zu reinigende Blut wie aus Figur 1 ersichtlich über die Arterienleitung 2 in einen Arterienleitungsbehälter 20, wobei es eine in der Arterienleitung 2 angeordnete Arterienleitungs-Schließeinrichtung 22 passiert. Von dem Arterienleitungsbehälter 20 wird das Blut nun weiter entlang der sich bis zu einer Blutbehandlungseinrichtung 5 erstreckenden Arterienleitung 2 gefördert. In diesem Ausführungsbeispiel erfolgt die Förderung des Blutes aus dem Arterienleitungsbehälter 20 mittels einer Pumpe 40, die beispielsweise als Rollenpumpe ausgebildet sein kann. In der Blutbehandlungseinrichtung 5 findet die eigentliche Reinigung des Blutes statt, indem über Diffusion und/oder Konvektion dem Blut toxische Substanzen und/oder Flüssigkeit entzogen wird. Die Reinigung des Blutes erfolgt hierbei über eine in der Blutbehandlungseinrichtung 5 angeordneten semipermeablen Membran 8. Das zu reinigende Blut fließt in diesem Ausführungsbeispiel im Gegenstrom zu einer über einen Dialysierflüssigkeitszulauf 6 der Blutbehandlungseinrichtung 5 zugeführten Dialysierflüssigkeit über die semipermeable Membran. Über einen an der Blutbehandlungseinrichtung 5 angeordneten Dialysierflüssigkeitsablauf 7 wird die mit den aus dem zu reinigenden Blut entfernten Toxinen angereicherte verbrauchte Dialysierflüssigkeit einer nichtdargestellten Sammeleinrichtung zugeführt. Das gereinigte Blut wird der Blutbehandlungseinrichtung 5 mittels einer angeordneten Venenleitung 3 entnommen und in einen Venenleitungsbehälter 30 gefördert. Von dort wird das gereinigte Blut über eine Fortsetzung der Venenleitung 3, wobei es eine in der Venenleitung 3 angeordnete Venenleitungs-Schließeinrichtung 32 passiert, wieder der Single-Lumen-Anordnung 10 zugeführt. Hier wird nun das gereinigte Blut über den Patientenzugang 12 des Y-Stücks 11 fast vollständig wieder in das Gefäßsystem 70 des zu therapierenden Patienten 1 reinfundiert.

Bei einer extrakorporalen Blutbehandlungsvorrichtung mit einer Single-Lumen-Anordnung 10 als Zugang zum Gefäßsystem 70 des Patienten 1 ist es allerdings aufgrund dieser speziellen Ausgestaltung nicht möglich, dem zu therapierenden Patienten 1 gleichzeitig sowohl zu reinigendes Blut zu entnehmen als auch gereinigtes Blut wieder in den Patienten 1 zu reinfundieren. Dazu sind extrakorporale Blutbehandlungsvorrichtungen mit Single-Lumen- Anordnung 10 wie in diesem Ausführungsbeispiel beschrieben mit speziellen Steuer- und Sensorelementen ausgestattet, die es ermöglichen phasenweise abwechselnd dem Patienten 1 zu reinigendes Blut zu entnehmen und gereinigtes Blut zu reinfundieren. Zu diesen Steuer und Sensorelementen gehören neben den bereits erwähnten Arterienleitungs- und Venenleitungs-Schließeinrichtungen 22, 32 und der Pumpe 40 dem Arterienleitungsbehälter 20 bzw. dem Venenleitungsbehälter 30 zugeordnete Sensoreinrichtungen 21, 31, durch die eine Aussage über den Füllstand in dem jeweiligen Behälter 20, 30 gewonnen werden kann. Weiterhin gehören dazu noch eine Steuereinrichtung 50, eine Überwachungseinrichtung 51, eine Speichereinrichtung 52, eine Anzeigeeinrichtung 60, eine Eingabeeinrichtung 61 sowie eine Kommunikationseinrichtung 62.

Die Kommunikationsrichtung dieser einzelnen Steuer- und Sensorelemente ist in Figur 1 durch Pfeile dargestellt. So empfängt die aus Steuereinrichtung 50, Überwachungseinrichtung 51 und Speichereinrichtung 52 bestehende Gruppe Daten von den Sensorelementen 31 und 21. Aufgrund dieser Daten können die Elemente dieser Gruppe und insbesondere die Steuereinrichtung 50 sowohl die Schließeinrichtungen 22 und 32 als auch die Pumpe 40 Anweisungen übermitteln, ihren aktuellen Betriebszustand zu ändern. Ferner können die Steuereinrichtung 50, die Überwachungseinrichtung 51 und die Speichereinrichtung 52 mit der Anzeigeeinrichtung 60, der Eingabeeinrichtung 61 sowie der Kommunikationseinrichtung 62 bidirektional Daten und Informationen austauschen.

Nachfolgend wird nun die Funktionsweise des Ausführungsbeispiels gemäß Figur 1 beschrieben, wobei nicht auf den Therapiebeginn Bezug genommen wird, sondern auf die Arbeitsweise während einer schon laufenden Therapie. Beginnend mit einer arteriellen Phase stellt sich die Situation im normalen störungsfreien Betrieb wie folgt dar:

Während der arteriellen Phase ist die Arterienleitungs-Schließeinrichtung 22 geöffnet, sodass das dem Patienten 1 mittels der Single-Lumen-Anordnung 10 entnommene Blut durch die Arterienleitung 2 in den Arterienleitungsbehälter 20 fließen kann. Am Anfang einer arteriellen Phase herrscht ein Unterdruck in dem Arterienleitungsbehälter 20, welcher dafür sorgt, dass das Blut vom Patienten angesogen wird und sich der Arterienleitungsbehälter 20 bis zu einem bestimmten Füllstand füllt, wo ein Druckausgleich zwischen dem Unterdruck in dem Arterienleitungsbehälter 20 und dem Blutdruck des Patienten stattgefunden hat. Ab diesem Zeitpunkt füllt sich der Arterienleitungsbehälter 20 nicht weiter und es fließt Blut in dem Maße nach, wie es durch die Pumpe (40) aus dem Arterienleitungsbehälter 20 gefördert wird. Gleichzeitig fördert die Pumpe 40 das bereits in dem Arterienleitungsbehälter 20 befindliche Blut weiter durch die Fortsetzung der Arterienleitung 2 in die als Dialysator ausgebildete Blutbehandlungseinrichtung 5, wo dem Blut toxische Substanzen mittels Diffusion und/oder Konvektion entzogen sowie das Gewicht des Patienten durch die mittels Ultrafiltration entzogene Flüssigkeit verringert wird. Von dort gelangt das nunmehr bereits behandelte Blut über die Venenleitung 3 in den Venenleitungsbehälter 30. Da in der arteriellen Phase die Venenleitungs-Schließeinrichtung 32 geschlossen ist, kann das im Venenleitungsbehälter 30 befindlich Blut nicht abfließen und über die Single-Lumen-Anordnung in das Gefäßsystem 70 des Patienten 1 reinfundiert werden. Aufgrund der geschlossenen Venenleitungs-Schließeinrichtung 32 füllt sich daher der Venenleitungsbehälter 30 mit bereits behandeltem Blut, bis ein bestimmter durch einen Grenzwert definierter oberer Füllstand im Venenleitungsbehälter 30 erreicht ist. Dieser Grenzwert wird im vorliegenden Ausführungsbeispiel gemäß Figur 1 durch die als Drucksensor ausgebildete Sensoreinrichtung 31 anhand eines vordefinierten Überdruckes innerhalb des Venenleitungsbehälters 30 erfasst. Wird dieser Überdruckwert durch den Drucksensor 32 an die Steuereinrichtung 50 übermittelt, veranlasst diese gleichzeitig (Signal A) ein Schließen der Arterienleitung 2 durch die Arterienleitungs-Schließeinrichtung 22 und ein Öffnen der Venenleitung 3 durch die Venenleitungs-Schließeinrichtung 32.

Damit ist die arterielle Phase beendet und es beginnt die venöse Phase, in welcher dem Patienten 1 bereits behandeltes Blut reinfundiert wird. Dies geschieht am Anfang einer jeden venösen Phase durch den im Venenleitungsbehälters 30 aufgebauten Überdruck bis zu einem Zeitpunkt, wo ein Druckausgleich zwischen dem Blutdruck des Patienten und dem Überdruck im Venenleitungsbehälters 30 stattgefunden hat. Ab diesem Zeitpunkt entleert sich der Venenleitungsbehälters 30 nur noch in dem Maße, wie die Pumpe (40) neues gereinigtes Blut in den Venenleitungsbehälters 30 pumpt. In der venösen Phase ist die Venenleitung 3 nicht mehr durch die Venenleitungs-Schließeinrichtung 32 geschlossen, so dass das in dem Venenleitungsbehälter 30 befindlich bereits behandelte Blut in das Gefäßsystem 70 des Patienten reinfundiert wird. Der Venenleitungsbehälter 30 entleert sich dabei aufgrund des Überdruckes der im Venenleitungsbehälter 30 vorliegt und der andauernden Förderung des Blutes durch die Pumpe 40 in den Venenleitungsbehälter 30. Unter Normalbedingungen (keine Störung in der Venenleitung) ist der Abfluß aus dem Venenleitungsbehälter 30 größer als der Zufluss in den Venenleitungsbehälter 30. Durch die Förderung der Pumpe 40 wird nicht nur der Venenleitungsbehälter 30 kontinuierlich weiter mit Blut beliefert, sondern auch der Arterienleitungsbehälter 20 entleert. Dies geschieht solange bis ein bestimmter durch einen Grenzwert definierter unterer Füllstand im Arterienleitungsbehälter 20 erreicht ist. Dieser Grenzwert wird im vorliegenden Ausführungsbeispiel gemäß Figur 1 durch die als Drucksensor ausgebildete Sensoreinrichtung 21 anhand eines vordefinierten Unterdruckes innerhalb des Arterienleitungsbehälters 20 erfasst. Wird dieser Unterdruckwert durch den Drucksensor 21 an die Steuereinrichtung 50 übermittelt veranlasst diese gleichzeitig (Signal B) ein Schließen der Venenleitung 3 durch die Venenleitungs-Schließeinrichtung 32 und ein Öffnen der Arterienleitung durch die Arterienleitungs-Schließeinrichtung 22.

Damit ist nun auch die venöse Phase beendet und während dieser aus arterieller und venöser Phase bestehenden Phase hat die Pumpe 40 im wesentlichen innerhalb bestimmter Toleranzgrenzen das Phasenvolumen $V_{ist} = V_{soll}$ gefördert. Diese bis jetzt beschriebene Funktionsweise stellt die gewünschte Funktionsweise ohne Störung dar. Bei Beginn der nächsten Phase bestehend aus arterieller und venöser Phase befindet sich im Patientenzugang 12 des Y-Stücks 11 (Figur 2) der Single-Lumen-Anordnung 10 bereits behandeltes Blut, welches nun in der arteriellen Phase wieder dem Reinigungskreislauf in der extrakorporalen Blutreinigungsvorrichtung zugeführt wird, ohne dass es zuvor dem Patienten 1 wieder reinfundiert wurde. Man bezeichnet das Volumen des Patientenzugangs 12 daher auch als Totvolumen 71, da bei einer extrakorporalen Blutreinigungsvorrichtung mit einer Single-Lumen-Anordnung 4 es unvermeidbar ist, dass das bei Beendigung der venösen Phase sich in diesem Totvolumen 71 befindliche Blut, ohne zuvor in das Gefäßsystem 70 des Patienten 1 reinfundiert worden zu sein, nochmals einem Reinigungszyklus unterworfen wird.

Es ist daher von besonderem Interesse, das Verhältnis von Phasenvolumen $V_{ist}$, zu dem Totvolumen (71) $V_{Tot}$ möglichst groß bzw. möglichst nah an dem Wert $V_{soll}/V_{Tot}$ zu halten. Deshalb ist es wünschenswert, dass bei auftretenden Störungen, besonders im Schlauchsystem der extrakorporalen Blutreinigungsvorrichtung die Vorrichtung möglichst schnell selbsttätig entsprechende Aktionen ausführt. Nachfolgend soll die selbsttätige Regulierung dieses Ausführungsbeispiels anhand einer auf einem Knick in der Venenleitung 3 zwischen dem Y-Stück 11 und dem Venenleitungsbehälter 30 basierenden Störung im Schlauchsystem der extrakorporalen Blutreinigungsvorrichtung beginnend während einer venösen Phase beschrieben werden.

Durch den Knick an dieser Stelle in der Venenleitung 3 erhöht sich dort der Flusswiderstand für das Blut, dass aus dem Venenleitungsbehälter 30 entleert wird und strömt somit während der venösen Phase langsamer als ohne Knick. Der Abfluß aus dem Venenleitungsbehälter 30 ist bei einer Störung, auf welche das System automatisch reagieren kann, langsamer als ohne die Störung jedoch noch immer schneller als der durch die Pumpe bestimmte Zufluß. Somit verschiebt sich das Verhältnis aus Ab- und Zufluss aus dem Venenleitungsbehälter 30 zugunsten des Zuflusses, wodurch das Flüssigkeitsvolumen im Venenleitungsbehälter 30 nicht ab-, sondern zunimmt. Gleichzeitig wird aber der Arterienleitungsbehälter 20 bei geschlossener Arterienleitungs-Schließeinrichtung 22 durch die andauernde Förderung der Pumpe 40 weiter entleert bis der vordefinierte Grenzwert für den Unterdruck in dem Arterienleitungsbehälter 20 erreicht ist. Nun wird durch die Steuereinrichtung 50 die Arterienleitungs-Schließeinrichtung 22 geöffnet und die Venenleitungs-Schließeinrichtung 32 geschlossen, so dass die venöse Phase beendet ist und die arterielle Phase beginnt. Dadurch, dass während der venösen Phase durch den Knick eine langsamere Entleerung des Venenleitungsbehälters 30 als im ungestörten System stattgefunden hat, ist der Füllstand dort zu Beginn der arteriellen Phase im Vergleich zum ungestörten extrakorporalen Kreislauf höher, so dass bei unveränderter Förderrate der Pumpe 40 der vordefinierte Grenzwert für den Überdruck in dem Venenleitungsbehälter 30 schneller erreicht wird. Dies bedeutet, dass das arterielle Phasenvolumen im Vergleich zum ungestörten System gering ist, da die Steuereinrichtung 50 die arterielle Phase beendet, wenn die Sensoreinrichtung 31 den vordefinierten Grenzwert für den Überdruck in dem Venenleitungsbehälter 30 erfasst und an die Steuereinrichtung 50 weiterleitet. In der darauf folgenden venösen Phase wiederholt sich dieses Szenario, bei anhaltender Störung. Dadurch dass die Entleerung des Venenleitungsbehälters 30 weiterhin verlangsamt erfolgt, ist der Füllstand im Venenleitungsbehälters 30 am Ende dieser venösen Phase noch hoher als in der Phase zuvor, so dass in der nun folgenden arteriellen Phase noch weniger Blut durch die Pumpe 40 in den Venenleitungsbehälter 30 gefördert würde. Ohne ein Eingreifen der erfindungsgemäßen Regelung würde das System nun eskalieren und im Extremfall wären sowohl im Venenleitungsbehälter 30 als auch im Arterienleitungsbehälter 20 die vordefinierten Grenzwerte sofort nach Umschalten durch die Steuereinrichtung 50 von der arteriellen in die venöse Phase oder umgekehrt erreicht und es würde kein Phasenvolumen mehr gefördert. Um dem entgegenzuwirken wird mit Hilfe der Steuer-, Überwachungs- und Speichereinrichtungen 50, 51 und 52 die Abweichung $\Delta V$ des Istwertes $V_{ist}$ des durch die Pumpe 40 während einer aus arterieller und venöser Phase gebildeten Phase geförderten Phasenvolumens von dem in der Speichereinrichtung 52 abgelegten Sollwert $V_{soll}$ bestimmt. Im vorliegenden Fall ist die Abweichung $\Delta V$ positiv und sofern sie außerhalb eines vordefinierten Toleranzbereiches liegt, greift nun der Regelkreis in das System ein und veranlasst die Steuereinrichtung 50 dazu, die Förderrate der Pumpe 40 um einen bestimmten Betrag zu senken. Durch die nunmehr geringere Förderrate der Pumpe 40 wird während einer venösen Phase nunmehr weniger Blutvolumen in den Venenleitungsbehälter 30 gefördert, so dass - ggf. erst nach mehrerer aus arterieller und venöser Phase bestehenden Zyklen bzw. Phasen - das durch die Entleerung des Venenleitungsbehälters 30 während einer venösen Phase definierte Blutvolumen größer ist als das in dieser venösen Phase durch die Pumpe 40 in den Venenleitungsbehälter 30 geförderte Blutvolumen. Durch diese Maßnahme erhöhen sich die absoluten Zeiten für die venöse und arterielle Phase, so dass obwohl die Förderrate der Pumpe 40 gesenkt wird, das in einer Phase bestehend aus arterieller und venöser Phase durch die Pumpe 40 geförderte Blutvolumen und damit das Phasenvolumen $V_{ist}$ erhöht wird.

Befindet sich das Phasenvolumen $V_{ist}$, wieder im Toleranzbereich von $V_{soll}$ wirkt der Regler nicht mehr. Das Phasenvolumen $V_{ist}$ wurde somit durch den Regelkreis selbsttätig ohne Einschreiten des Anwenders in den Toleranzbereich des Sollwerts $V_{soll}$ eingestellt.

Wird die Störung - vorliegender Knick in der Venenleitung 2 - entfernt greift auch dann der Regelkreis wieder ein. Der nunmehr geringerer Flusswiderstand hätte eine Erhöhung des Phasenvolumens zur Folge, so dass die Steuereinrichtung 50 durch den Regelkreis die Förderrate der Pumpe 40 erhöhen würde, bis das Phasenvolumen $V_{ist}$ wieder im Toleranzbereich des Sollwertes $V_{soll}$ liegt.

Somit ist es möglich, die Therapie mit der erfindungsgemäßen Vorrichtung aufrecht zu erhalten, solange im extrakorporalen Blutkreislauf noch eine Zirkulation stattfindet und die Störung das Schlauchsystem nicht komplett blockiert.

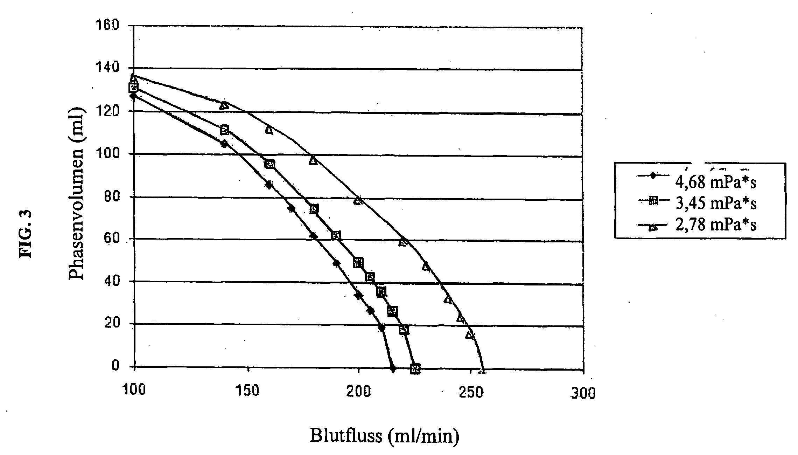

Als eine Störung im weiteren Sinn kann auch eine Änderung der Viskosität des Blutes des Patienten 1 während einer Therapie angesehen werden. Wie in Figur 3 dargestellt verhält sich nämlich für verschiedene Viskositäten das Phasenvolumen zum Blutfluss unterschiedlich. Viskositätsänderungen während einer Therapie treten natürlich auch durch den nicht zu vermeidenden Flüssigkeitsverlust des Patienten 1 auf. Auch solche Viskositätsänderungen verursachen eine Änderung des Flusswiderstandes im Schlauchsystem der Vorrichtung, die als Störung aufgefasst werden, da dadurch der Betrag des Verhältnisses von $V_{ist} / V_{Tot}$ verkleinert wird und somit die Effektivität der Therapie verschlechtert wird. Auch eine solche durch Viskositätsänderung des Blutes während der Therapie des Patienten 1 auftretende Störung kann durch den Regelkreis entsprechend beeinflusst werden, so dass die optimale Effektivität der Therapie erreicht wird.

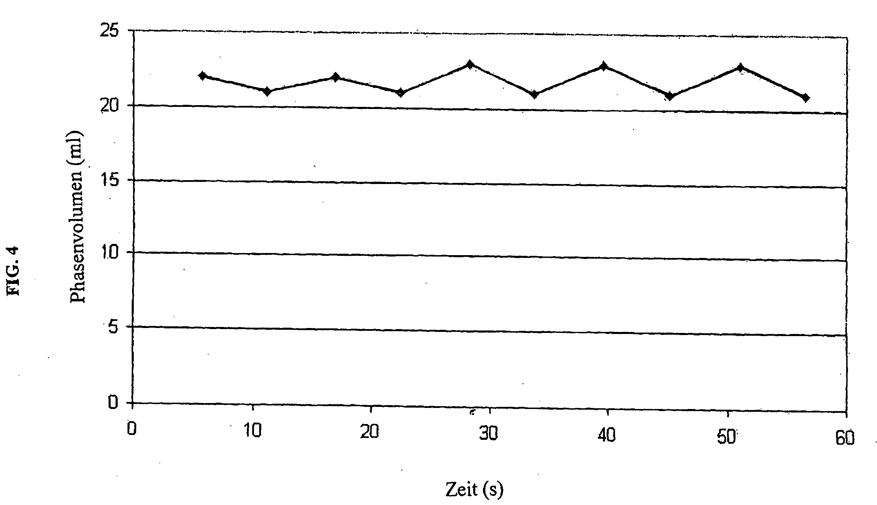

In Figur 4 ist beispielhaft das Verhalten des Phasenvolumens $V_{ist}$ im ungestörten Zustand über die Zeit bei Verwendung einer im Bereich der extrakorporalen Blutbehandlung üblichen Rollenpumpe 40 dargestellt. Die dort gezeigten Schwankungen des Phasenvolumens $V_{ist}$, die durch die Konstruktion und die physikalischen Gegebenheiten einer Rollenpumpe 40 bedingt sind, sind der Grund dafür, dass nicht auf einen exakten Sollwert $V_{soll}$ geregelt wird, sondern auf einen Toleranzbereich oder Tunnel um einen solchen Sollwert. Der Hintergrund hierfür ist, dass durch die Verwendung einer Rollenpumpe Abweichungen des Phasenvolumens $V_{ist}$ unvermeidlich sind. Solche Abweichungen sollen aber nicht bereits dazu führen, dass das Phasenvolumen $V_{ist}$ auf den Sollwert $V_{soll}$ geregelt wird. Erst wenn der Istwert des Phasenvolumens $V_{ist}$ den vorgegebenen Toleranzbereich bzw. Tunnel verlässt, soll der Regelkreis eingreifen, um die bestmögliche Effektivität der Therapie bei bestmöglicher Wirtschaftlichkeit zu gewährleisten.

Bezugszeichenliste:

1 - Patient
2 - Arterienleitung
3 - Venenleitung
5 - Blutbehandlungseinrichtung
6 - Dialysierflüssigkeitszulauf
7 - Dialysierflüssigkeitsablauf
8 - Membran
10 - Single-Lumen-Anordnung
11 - Y-Stück
12 - Patientenzugang
20 - Arterienleitungsbehälter
21 - Sensoreinrichtung
22 - Arterienleitungs-Schließeinrichtung
30 - Venenleitungsbehälter
31 - Sensoreinrichtung
32 - Venenleitungs-Schließeinrichtung
40 - Pumpe
50 - Steuereinrichtung
5 1 - Überwachungseinrichtung
52 - Speichereinrichtung
60 - Anzeigeeinrichtung
61 - Eingabeeinrichtung
62 - Kommunikationseinrichtung
70 - Gefäßsystem
71 - Totvolumen

**Patentansprüche**

1. Extrakorporale Blutbehandlungsvorrichtung mit

- einer Steuereinrichtung (50) zur Steuerung des Öffnens und Schließens einer Arterienleitung (2) bei gleichzeitigem Schließen und Öffnen einer Venenleitung (3) in einer Single-Lumen-Anordnung (10), mit welcher unbehandeltes Blut durch die Single-Lumen-Anordnung (10) und die Arterienleitung (2) abgezogen, durch eine Blutbehandlungseinrichtung (5) geleitet und behandeltes Blut durch die Venenleitung (3) und die Single-Lumen-Anordnung (10) zurückgeführt wird,
- einem Arterienleitungsbehälter (20), der in der Arterienleitung (2) vor der Blutbehandlungseinrichtung (5) angeordnet ist,
- einem Venenleitungsbehälter (30), der in der Venenleitung (3) hinter der Blutbehandlungseinrichtung (5) angeordnet ist,
- einer Sensoreinrichtung (31) zum Erfassen einer über einem Grenzwert liegenden Menge behandelten Bluts in dem Venenleitungsbehälter (30),

- einer Sensoreinrichtung (21) zum Erfassen einer unter einem Grenzwert liegenden Menge unbehandelten Bluts in dem Arterienleitungsbehälter (20),

- einer Arterienleitungs-Schließeinrichtung (22) zum Verschließen der Arterienleitung (2) bei gleichzeitigem Öffnen der Venenleitung (3) ansprechend auf ein Signal A, das von der Sensoreinrichtung (31) an die Arterienleitungs-Schließeinrichtung (22) übermittelt wird,

- einer Venenleitungs-Schließeinrichtung (32) zum Verschließen der Venenleitung (3) bei gleichzeitigem Öffnen der Arterienleitung (2) ansprechend auf ein Signal B, das von der Sensoreinrichtung (21) an eine Venenleitungs-Schließeinrichtung (32) übermittelt wird und

- wenigstens einer Pumpe (40) zur Förderung eines Phasenvolumens $V_{Phase}$ mit einer Förderrate $Q_{BP}$,

**dadurch gekennzeichnet, dass**

die Steuerung (50) dann, wenn das tatsächlich geförderte Phasenvolumen $V_{ist}$ von dem Sollwert des Phasenvolumens $V_{soll}$ abweicht, die Förderrate $Q_Bp$ der wenigstens einen Pumpe (40) so regelt, dass diese Abweichung $\Delta V$ gegen Null geht, wobei $V_{soll}$ für die erste Phase bei Beginn der Dialyse vorgegeben wird und für jede weitere Phase dem tatsächlich geförderten Phasenvolumen $V_{ist}$ der vorhergehenden Phase entspricht.

2. Vorrichtung nach Anspruch 1, **dadurch gekennzeichnet, dass** die Steuereinrichtung (50) als Regelkreis ausgebildet ist, bei dem die selbsttätige Angleichung des Istwertes $V_{ist}$ des Phasenvolumens an dessen Sollwert $V_{soll}$ und der einhergehenden Änderung der Zeitspanne einer Phase $tp_{hase}$ in der venösen Phase als auch in der arteriellen Phase erfolgen kann.

3. Vorrichtung nach Anspruch 1, **dadurch gekennzeichnet, dass** der Regler des Regelkreises als P-Regler, I-Regler, D-Regler, PI-Regler, PD-Regler, PID-Regler, Zweipunktregler, Dreipunktregler, Mehrpunktregler bzw. Fuzzy-Regler ausgebildet ist.

4. Vorrichtung nach einem der Ansprüche 1 oder 2, **dadurch gekennzeichnet, dass** der Sollwert $V_{soll}$ des Phasenvolumens mindestens fünfmal, vorzugsweise mindestens zehnmal so groß ist wie ein Totvolumen $V_{Tot}$ (71) der Single-Lumen-Anordnung (10).

5. Vorrichtung nach Anspruch 1, **dadurch gekennzeichnet, dass** die Steuereinrichtung (50) derart ausgebildet ist, dass sie die Regelung auf das Phasenvolumen unmittelbar nach Detektion einer Abweichung $\Delta V \neq 0$ des Istwertes $V_{ist}$ des Phasenvolumens von dessen Sollwert $V_{soll}$ um eine vorgegebenen Totzeit $T_{Tot}$ verzögert.

6. Vorrichtung nach Anspruch 4, **dadurch gekennzeichnet, dass** die vorgegebenen Totzeit $T_{Tot}$ wenigstens zwei Phasen, bevorzugt wenigsten vier Phasen und maximal zehn Phasen beträgt,

7. Vorrichtung nach einem der vorherigen Ansprüche, **dadurch gekennzeichnet, dass** die wenigstens eine Pumpe (40) zwischen dem Arterienleitungsbehälter (20) und dem Venenleitungsbehälter (30) angeordnet ist.

8. Vorrichtung nach einem der vorherigen Ansprüche, **dadurch gekennzeichnet, dass** die Sensoreinrichtungen (21, 31) als Drucksensoren ausgebildet sind.

9. Vorrichtung nach einem der vorherigen Ansprüche, **dadurch gekennzeichnet, dass** der durch die Sensoreinrichtung (31) gemessene Überdruck im Venenleitungsbehälter (30) ein Verschließen der Arterienleitungs-Schließeinrichtung (22) und ein Öffnen der Venenleitungs-Schließeinrichtung (32) auslöst und dass der durch die Sensoreinrichtung (21) gemessene Unterdruck im Arterienleitungsbehälter (20) ein Verschließen der Venenleitungs-Schließeinrichtung (32) und ein Öffnen der Arterienleitungs-Schließeinrichtung (22) auslöst

10. Vorrichtung nach einem der vorherigen Ansprüche, **dadurch gekennzeichnet, dass** zur Bestimmung des Istwertes $V_{ist}$ des Phasenvolumens die Förderrate $Q_Bp$ der Pumpe (40) sowie die Zeitspanne einer Phase $t_{Phase}$ verwendet wird, wobei die Zeitspanne einer Phase $t_{Phase}$ definiert ist als Summe der Zeitspanne $t_{Ven\_Phase}$, die zwischen dem Signal A und dem Signal B liegt, die von der Steuereinrichtung (50) an die Arterienleitungs-Schließeinrichtung (22) und die Venenleitungs-Schließeinrichtung (32) übermittelt werden, und der Zeitspanne $t_{Art\_Phase}$, die zwischen dem Signal B und dem Signal A liegt, die von der Steuereinrichtung (50) an die Arterienleitungs-Schließeinrichtung (22) und die Venenleitungs-Schließeinrichtung (32) übermittelt werden.

**Claims**

1. Extracorporeal blood treatment apparatus with

   - a control device (50) for controlling opening and closing of an arterial line (2) with simultaneous closing and opening of a venous line (3) in a single-lumen arrangement (10), with which untreated blood is taken through the single-lumen arrangement (10) and the arterial line (2), passed through a blood treatment device (5) and treated blood is fed back through the venous line (3) and the single-lumen-arrangement (10),
   - an arterial line container (20) which is arranged in the arterial line (2) in front of the blood treatment device (5),
   - a venous line container (30) which is arranged in the venous line (3) following the blood treatment device (5),
   - a sensor device (31) for detecting an amount of treated blood being above a limit value in the venous line container (30),
   - a sensor device (21) for detecting an amount of untreated blood being below a limit value in the arterial line container (20),
   - an arterial line closure device (22) for closing the arterial line (2) with simultaneous opening of the venous line (3) in response to a signal A which is transmitted by the sensor device (31) to the arterial line closure device (22),
   - a venous line closure device (32) for closing the venous line (3) with simultaneous opening of the arterial line (2) in response to a signal B which is transmitted by the sensor device (21) to a venous line closure device (32), and
   - at least one pump (40) for conveying a phase volume $V_{Phase}$ with a pumping rate $Q_{BP}$,

   **characterized in that**
   if the actually conveyed actual value $V_{act}$ of the phase volume deviates from the nominal value $V_{nom}$ of the phase volume, the control device (50) regulates the pumping rate $Q_{BP}$ of the at least one pump (40) so that this deviation $\Delta V$ tends to be nil, wherein $V_{nom}$ is predetermined for the first phase at the beginning of the dialysis and corresponds for each further phase to the actually conveyed actual value $V_{act}$ of the phase volume of the preceding phase.

2. Apparatus according to claim 1, **characterized in that** the control device (50) is designed as control circuit with which the automatic adjustment of the actual value $V_{act}$ of the phase volume to its nominal value $V_{nom}$ and the associated change of the time period of a phase $t_{Phase}$ can be performed in the venous phase as well as in the arterial phase.

3. Apparatus according to claim 1, **characterized in that** the controller of the control circuit is designed as P-controller, I-controller, D-controller, PI-controller, PDcontroller, PID-controller, two-point controller, three-point controller, multi-point controller or fuzzy-controller.

4. Apparatus according to any one of claims 1 or 2, **characterized in that** the nominal value $V_{nom}$ of the phase volume is at least 5 times, preferably at least 10 times, larger as a dead volume $V_{dead}$ (71) of the single-lumen arrangement (10).

5. Apparatus according to claim 1, **characterized in that** the control device (50) is designed in such a manner that it delays the regulation to the phase volume directly after detection of a deviation $\Delta V \neq 0$ of the actual value $V_{act}$ of the phase volume from its nominal value $V_{nom}$ by a predetermined dead time $T_{dead}$.

6. Apparatus according to claim 4, **characterized in that** the predetermined dead time $T_{dead}$ is at least two phases, preferably at least four phases, and maximum 10 phases.

7. Apparatus according to any one of the preceding claims, **characterized in that** the at least one pump (40) is arranged between the arterial line container (20) and the venous line container (30).

8. Apparatus according to any one of the preceding claims, **characterized in that** the sensor devices (21, 31) are designed as pressure sensors.

9. Apparatus according to any one of the preceding claims, **characterized in that** the over-pressure in the venous line container (30) measured by the sensor device (31) triggers closing of the arterial line closure device (22) and opening of the venous line closure device (32) and that the under-pressure in the arterial line container (20) measured by the sensor device (21) triggers closing of the venous line closure device (32) and opening of the arterial line closure device (22).

**10.** Apparatus according to any one of the preceding claims, **characterized in that** for determination of the actual value $V_{act}$ of the phase volume, the pumping rate $Q_{BP}$ of the pump (40) as well as the time period of a phase $t_{Phase}$ is used, wherein the time period of a phase $t_{Phase}$ is defined as sum of the time period $t_{Ven-Phase}$ which is between signal A and signal B, which are transmitted by the control device (50) to the arterial line closure device (22) and the venous line closure device (32), and the time period $t_{Art-Phase}$, which is between signal B and signal A which are transmitted by the control device (50) to the arterial line closure device (22) and the venous line closure device (32).

**Revendications**

**1.** Dispositif de traitement de sang extracorporel comprenant

- un système de commande (50) servant à commander l'ouverture et la fermeture d'une conduite artérielle (2) tout en fermant et en ouvrant dans le même temps une conduite veineuse (3) dans un ensemble monolumière (10), à l'aide duquel le sang non traité est prélevé par l'ensemble monolumière (10) et la conduite artérielle (2), est acheminé par un système de traitement de sang (5) et à l'aide duquel le sang traité est ramené par la conduite veineuse (3) et l'ensemble monolumière (10),
- un récipient de conduite artérielle (20), qui est disposé dans la conduite artérielle (2) devant le système de traitement de sang (5),
- un récipient de conduite veineuse (30), qui est disposé dans la conduite veineuse (3) derrière le système de traitement de sang (5),
- un système capteur (31) servant à relever une quantité de sang traité supérieure à une valeur limite dans le récipient de conduite veineuse (30),
- un système capteur (21) servant à relever une quantité de sang non traité inférieure à une valeur limite dans le récipient de conduite artérielle (20),
- un système de fermeture de conduite artérielle (22) servant à fermer la conduite artérielle (2) tout en ouvrant dans le même temps la conduite veineuse (3) en réponse à un signal A, qui est transmis par le système capteur (31) au système de fermeture de conduite artérielle (22),
- un système de fermeture de conduite veineuse (32) servant à fermer la conduite veineuse (3) tout en ouvrant dans le même temps la conduite artérielle (2) en réponse à un signal B, qui est transmis par le système capteur (21) à un système de fermeture de conduite veineuse (32), et
- au moins une pompe (40) servant à refouler un volume de phase $V_{phase}$ à un débit de refoulement $Q_{BP}$,

**caractérisé en ce que**
la commande (50) régule, quand le volume de phase réellement refoulé $V_{réel}$ s'écarte de la valeur théorique du volume de phase $V_{théorique}$, le débit de refoulement $Q_{BP}$ de la pompe (40) au moins au nombre de une de sorte que ledit écart $\Delta V$ tend vers zéro, $V_{théorique}$ étant spécifié pour la première phase au début de la dialyse et correspondant, pour chaque phase supplémentaire, au volume de phase réellement refoulé $V_{réel}$ de la phase qui précède.

**2.** Dispositif selon la revendication 1, **caractérisé en ce que** le système de commande (50) est réalisé sous la forme d'un circuit de régulation, dans lequel l'alignement autonome de la valeur réelle $V_{réel}$ du volume de phase sur la valeur théorique de ce dernier $V_{théorique}$ et la modification allant de pair du laps de temps d'une phase $t_{phase}$ peuvent être effectués dans la phase veineuse tout comme dans la phase artérielle.

**3.** Dispositif selon la revendication 1, **caractérisé en ce que** le régulateur du circuit de régulation est réalisé sous la forme d'un régulateur P, d'un régulateur I, d'un régulateur D, d'un régulateur PI, d'un régulateur PD, d'un régulateur PID, d'un régulateur à deux positions, d'un régulateur à trois positions, d'un régulateur à plusieurs positions ou d'un régulateur à logique floue.

**4.** Dispositif selon l'une quelconque des revendications 1 ou 2, **caractérisé en ce que** la valeur théorique $V_{théorique}$ du volume de phase est au moins cinq fois, de préférence au moins dix fois plus grande qu'un volume mort $V_{mort}$ (71) de l'ensemble monolumière (10).

**5.** Dispositif selon la revendication 1, **caractérisé en ce que** le système de commande (50) est réalisé de telle manière qu'il retarde la régulation sur le volume de phase directement après la détection d'un écart $\Delta V \neq 0$ de la valeur réelle $V_{réel}$ du volume de phase par rapport à la valeur théorique $V_{théorique}$ de ce dernier d'un temps mort $T_{mort}$ spécifié.

**6.** Dispositif selon la revendication 4, **caractérisé en ce que** le temps mort $T_{mort}$ spécifié représente au moins deux phases, de manière préférée au moins quatre phases et au maximum dix phases.

7. Dispositif selon l'une quelconque des revendications précédentes, **caractérisé en ce que** la pompe (40) au moins au nombre de une est disposée entre le récipient de conduite artérielle (20) et le récipient de conduite veineuse (30).

8. Dispositif selon l'une quelconque des revendications précédentes, **caractérisé en ce que** les systèmes capteurs (21, 31) sont réalisés sous la forme de capteurs de pression.

9. Dispositif selon l'une quelconque des revendications précédentes, **caractérisé en ce que** la surpression mesurée par le système capteur (31) dans le récipient de conduite veineuse (30) déclenche une fermeture du système de fermeture de conduite artérielle (22) et une ouverture du système de fermeture de conduite veineuse (32), et **en ce que** la dépression mesurée par le système capteur (21) dans le récipient de conduite artérielle (20) déclenche une fermeture du système de fermeture de conduite veineuse (32) et une ouverture du système de fermeture de conduite artérielle (22).

10. Dispositif selon l'une quelconque des revendications précédentes, **caractérisé en ce que** le débit de refoulement $Q_{BP}$ de la pompe (40) ainsi que le laps de temps d'une phase $t_{phase}$ sont utilisés aux fins de la détermination de la valeur réelle $V_{réel}$ du volume de phase, le laps de temps d'une phase $t_{phase}$ étant défini comme la somme du laps de temps $t_{phase-vein}$, qui se trouve entre le signal A et le signal B, qui sont transmis par le système de commande (50) au système de fermeture de conduite artérielle (22) et au système de fermeture de conduite veineuse (32), et du laps de temps $t_{phase\_art}$, qui se trouve entre le signal B et le signal A, qui sont transmis par le système de commande (50) au système de fermeture de conduite artérielle (22) et au système de fermeture de conduite veineuse (32).

**FIG. 1**

**FIG. 2**

FIG. 3

Phasenvolumen (ml)

Blutfluss (ml/min)

4,68 mPa*s
3,45 mPa*s
2,78 mPa*s

FIG. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3422375 A1
- US 4643714 A
- US 5318511 A
- US 518511 A
- DE 3422375 A
- US 4596550 A